# EUROPÄISCHE PATENTSCHRIFT

(11) **EP 2 064 236 B1**
(45) Veröffentlichungstag und Bekanntmachung des Hinweises auf die Patenterteilung: **02.10.2013**
(21) Anmeldenummer: 07802048.4
(22) Anmeldetag: 31.08.2007
(51) Int. Cl.: C07K 14/705

(54) **VERFAHREN ZUR VERBESSERUNG DER SPEZIFISCHEN EFFEKTORFUNKTION VON EINZELKETTEN- ANTIGEN-ERKENNENDEN GENETISCHEN KONSTRUKTEN (SCARC) DURCH DEREN MURINISIERUNG**
METHOD FOR IMPROVING THE SPECIFIC EFFECTOR FUNCTION OF SINGLE-CHAIN ANTIGEN-RECOGNIZING GENETIC CONSTRUCTS (SCARC) THROUGH MURINIZATION THEREOF
PROCÉDÉ D'AMÉLIORATION DE LA FONCTION D'EFFECTEUR SPÉCIFIQUE DE CONSTRUCTIONS GÉNÉTIQUES RECONNAISSANT UN ANTIGÈNE À CHAÎNE SIMPLE (SCARC) PAR LEUR MURINISATION

(30) Priorität: 04.09.2006 DE 102006041455
(43) Veröffentlichungstag der Anmeldung: 03.06.2009
(73) Patentinhaber: Johannes-Gutenberg-Universität Mainz, 55122 Mainz (DE)
(72) Erfinder: VOSS, Ralf-Holger, 55218 Ingelheim (DE); THEOBALD, Matthias, 55252 Mainz-Kastel (DE); INTAN, Ratna, Sari, 55118 Mainz (DE); ENGEL, Renate, 66636 Tholey (DE); THOMAS, Simone, 55128 Mainz (DE)
(74) Vertreter: Krauss, Jan
(86) Internationale Anmeldenummer: PCT/EP2007/007631
(87) Internationale Veröffentlichungsnummer: WO 2008/028601

(56) Entgegenhaltungen:
- WO-A-2004/056845
- VOSS RALF-HOLGER ET AL: "DESIGNING TCR FOR CANCER IMMUNOTHERAPY" METHODS IN MOLECULAR MEDICINE, HUMANA PRESS, TOTOWA, NJ, US, Bd. 109, 2005, Seiten 229-256, XP008069998 ISSN: 1543-1894
- VOSS R-H ET AL: "Tumor-specific murine T cell receptors displace endogenous TCRs in human T cells" CANCER CELL INTERNATIONAL, BIOMED CENTRAL, LONDON, GB, Bd. 4, Nr. Suppl 1, 1. Juli 2004 (2004-07-01), Seite S33, XP021007305 ISSN: 1475-2867
- PECORARI F ET AL: "Folding, heterodimeric association and specific peptide recognition of a murine alphabeta T-cell receptor expressed in Escherichia coli" JOURNAL OF MOLECULAR BIOLOGY, LONDON, GB, Bd. 285, Nr. 4, 29. Januar 1999 (1999-01-29), Seiten 1831-1843, XP004457383 ISSN: 0022-2836
- THOMAS SIMONE ET AL: "Playing the game together: Coexpression of a single chain T cell receptor and a T cell receptor constant-alpha domain triggers tumor reactivity." BLOOD, Bd. 108, Nr. 11, Part 1, November 2006 (2006-11), Seite 1061A, XP002474510 & 48TH ANNUAL MEETING OF THE AMERICAN-SOCIETY-OF-HEMATOLOGY; ORLANDO, FL, USA; DECEMBER 09 -12, 2006 ISSN: 0006-4971

## Beschreibung

Die vorliegende Erfindung betrifft ein Verfahren gemäß Anspruch 1 zur Herstellung einer humanen Zellinie, die einen funktionellen stabilisierten funktionellen humanen Einzelketten-TCR (scTCR) oder ein stabilisiertes humanes Antikörper-scFv-Fragment exprimiert. Bevorzugterweise handelt es sich bei den Einzelketten-TCR (scTCR) oder Antikörper-scFv-Fragmente, um scTCR oder Antikörper-scFv die tumorassoziierte Peptidantigene (TAA) erkennen. Die vorliegende Erfindung betrifft weiterhin einen mittels des Verfahrens der vorliegenden Erfindung rational mutierten gp100-Protein-spezifische T-Zell Antwort vermittelnden α/β T-Zell Rezeptoren und dessen Verwendungen.

In der Immuntherapie maligner Erkrankungen ist der adoptive T-Zell-Transfer gegenwärtig Gegenstand intensiver Forschung. Hierbei werden zum einen autologe Tumor-infiltrierende Lymphozyten eines Tumorpatienten isoliert, von denen eine Tumor-spezifische cytolytische Reaktivität angenommen wird, ex vivo massiv expandiert und dem Patienten reinfundiert. Da diese TILs zumeist geringer Reaktivität aufgrund der geringen Affinitäten des jeweiligen T-Zell-Rezeptors (TCR) oder anergetisierender Mechanismen sind, werden hoch-affine T-Zell-Rezeptoren über verschiedene Verfahren isoliert und entwickelt und diese durch z.B. retroviralen Gentransfer in autologe humane periphere Blutmonozyten (PBMC) eingeschleust.

Der native TZR ist ein membranständiger Heterodimer aus einer über eine Disulfidbrücke verbundenen alpha- und beta-Kette. Der TCR erkennt das im MHC-Molekül eingebettete Tumor-assoziierte Antigen (TAA) und initialisiert eine Signalkaskade, die in CD8+-T-Zellen die gegen die Ziel-Zelle gerichtete cytolytische Aktivität und in CD8+ und CD4+ Zellen eine accessorische, Zytokin-unterstützende Effektorfunktion auslöst. Die Zielzellen, wie etwa die Tumorzellen, werden in den programmierten Zelltod (Apoptose) getrieben.

Ein Hauptproblem besteht in der Möglichkeit, dass diese exogen eingeführten TCR mit den endogenen TCR interagieren können und ungewünschte Selbst-Reaktivitäten zeigen (1).

Um dies zu vermeiden wurden in der Vergangenheit Einzelketten-T-Zell-Rezeptoren entwickelt, in denen die variablen Domänen Valpha und Vbeta der beiden Ketten über einen verknüpfenden Aminosäure-Linker kovalent miteinander verbunden sind. Zumeist an der variablen Vbeta-Domäne wird die konstante Cbeta-Domäne verknüpft, an die wiederum ein signalgebendes Molekül wie z.B. die CD3-Zeta-Kette geknüpft wird, um dieses TCR-Konstrukt funktionell zu initiieren. Ein Nachteil dieser Konstrukte ist der beträchtliche chimäre Charakter, von dem nicht hinreichend gesagt werden kann, inwieweit die natürliche Signalgebung einer T-Zelle verändert wird.

Ein Lösungsweg zur Umgehung der ungewünschten Kettenpaarung ist das Design von Einzelketten-T-Zell-Rezeptoren. Dieses **scTCR-Konzept** dient dazu, ungewünschte Kettenpaarungen von endogenen zu exogenen Ketten zu verhindern. Solche Konstrukte sind gentechnisch beliebig konzipierbar und gewährleisten eine kovalente 1:1-Stöchiometrie der heterodimeren, variablen Domänen. Das scTCR-Konzept wurde ursprünglich für Antikörper entwickelt (2), konnte aber aufgrund der strukturellen Homologien zwischen Antikörpern und T-Zell-Rezeptoren auf letztere übertragen werden (3, 4). Hierbei werden die variablen Domänen über ein kurzes Peptid, einen "Linker", miteinander kovalent unter Wegfall der einen von beiden konstanten Domänen verknüpft. Solche Konstrukte sind gentechnisch beliebig konzipierbar und gewährleisten eine biochemisch gekoppelte 1:1-Stöchiometrie der heterodimeren, variablen Domänen.

Alternativ werden an die jeweiligen Ketten des heterodimeren Moleküls kurze Peptidsequenzen als Affinitäts-Anhängsel gentechnisch angefügt, die für eine spezifische Kettenpaarung Sorge tragen: hierzu wurden T-Zell-Rezeptoren 30 Aminosäure lange, carboxyterminale "tags", ein so genannter "leucine zipper", als Dimerisierungsmotiv angefügt (5). In der Vergangenheit zeigt sich, dass die Stabilität und Funktionalität der scTCR über die Kopplung an die Vollängen ζ-Kette des CD3-Komplexes erheblich verbessert werden konnte (4). Auch Doppelketten-TCR in Fusion zur CD3ζ-Kette werden eingesetzt. Statt der konstanten TCR-Domäne werden auch invariante Domänen funktionsfremder (Membran-) Proteine verwendet: CH2 / CH3 von Antikörpern (6), die Transmembrane des Korezeptors CD4 (7), die Membran-verankernde / Signal-gebende Domäne des FcεRIγ-Rezeptors (8). Eine Vielzahl der getesteten Konstrukte blieb hinter den Effizienzen und Spezifitäten ihrer wildtypischen Konstrukte (9).
Ein alternativer Weg ist die Beeinflussung der präferentiellen Kettenpaarung durch Einführen artifizieller Disulfidbrücken (10), birgt aber immer noch den Nachteil unerwünschter hybrider Kettenpaarung mit den endogenen Ketten aufgrund vorhandener sterischer Komplementarität.

Unter den TAA, die im Kontext von MHC-Klasse-I-Molekülen auf der Oberfläche von Tumorzellen präsentiert werden, sind die so genannten "universellen" TAA von besonderem Interesse. Diese TAA leiten sich überwiegend von zellulären Proteinen ab, die in normalen Zellen schwach exprimiert und in Tumorzellen überexprimiert werden. Zu diesen Proteinen gehört unter anderem das humane gp100-Protein, das z.B. in Melanomen, Glioblastomen oder colorektalem Krebs eine Rolle spielt (11).

Oligopeptide des gp100-Proteins können im Kontext mit MHC-Klasse-I-Molekülen auf der Zelloberfläche präsentiert werden und repräsentieren attraktive Zielstrukturen für CD8-positive T-Zellen. Das Ausmaß der T-Zell Antwort verläuft hierbei in einem definierten kinetischen Fenster (12). Der Komplex aus Peptid-MHC und TCR-CD3 multimerisiert, um eine effiziente Signaltransduktion zu bewirken, wobei die genaue Stöchiometrie und das Ausmaß der Oligomerisierung noch umstritten ist.

Wesentlicher Nachteil der scTCR-CD3ζ-Konstrukte ist die Notwendigkeit der Kopplung an das eigentlich signalgebende Molekül CD3ζ der T-Zelle. Dies könnte ein Risiko der T-Zell-Aktivierung darstellen, aus der allo-Reaktivitäten (Autoimmun-Reaktionen) entstehen. Zudem ist der chimäre Charakter der scTCR-CD3ζ-Ketten derart erhöht, dass Immunreaktionen gegen das Fremdprotein, insbesondere gegen den Fusionsbereich zur humanen CD3ζ-Kette zu befürchten sind.

DE 199 10 419 A1 beschreibt multivalente Proteine, die spezifisch für Zielzellen sind und die aus 3 Komponenten (a-c) bestehen, wobei (a) mindestens ein Wirkstoff, (b) ein oder mehrere Linker und (c) mindestens 2 Bindestrukturen sind. Bei den Bindestrukturen kann es sich beispielsweise um rekombinante Antikörper oder Teile davon aber auch Transmembran-Domänen handeln. Die multivalenten Proteine von DE 199 10 419 A1 können Signalsequenzen enthalten.

DE 695 22 216 T2 beschreibt Zielzellen-bindende chimäre Peptide, insbesondere rekombinante Antikörpermoleküle mit immundominanten Peptidsequenzen, wobei die immundominanten Peptidsequenzen von der Zielzelle internalisiert und prozessiert und anschließend präsentiert werden, was entweder zur Induktion oder zur Hemmung einer Immunreaktion führt. Derartige immundominante Peptide enthaltende Antikörpermoleküle können unter anderem durch Koexpression erhalten werden.

Novotny et al. 1991 (Novotny J, Ganju RK, Smiley ST, Hussey RE, Luther MA, Recny MA, Siliciano RF, Reinherz EL. A soluble, single-chain T-cell receptor fragment endowed with antigen-combining properties. Proc Natl Acad Sci U S A. 1991 Oct 1;88(19):8646-50) beschreibt Einzelketten-T-Zell-Rezeptoren (scTCR), die die beiden variablen Domänen eines TCR, Vₐₗₚₕₐ und V_{beta}, enthalten, die durch einen Peptidlinker verbunden sind. Außerdem enthalten die TCRs eine pelB-Sequenz für periplasmatische bakterielle Expression. Trotzdem wurden die scTCRs als unlösliches Protein in Form von inclusion bodies erhalten, zunächst gelöst und mittels HPLC gereinigt und anschließend mittels Glutathion rückgefaltet.

Plaksin et al. 1997 (Plaksin D, Polakova K, McPhie P, Margulies DH. A three-domain T cell receptor is biologically active and specifically stains cell surface MHC/peptide complexes. J Immunol. 1997 Mar 1;158(5):2218-27.) beschreibt ebenfalls einen scTCR, der aber aus 3 Domänen eines TCR, der spezifisch für ein HIV-gp120-abgeleitetes Peptid im Komplex mit Maus-MHC I, aufgebaut ist. Dabei ist die variable Domäne Valpha durch einen Peptid-Linker mit 2 Domänen der beta-Kette (Vbeta und Cbeta) verbunden. Diese 3-Domänen scTCRs wurden ebenfalls in E. coli in unlöslicher Form exprimiert, anschließend aus den inclusion bodies gelöst und über ein aufwendiges und langwieriges Verfahren mittels Glutathion rückgefaltet. Der scTCR zeigte eine moderate Affinität (im µM-Bereich) gegenüber dem spezifischen Peptid/MHC I-Komplex.

Weiter sind Techniken im Stand der Technik bekannt, wobei murine Antikörper humanisiert werden, d.h. Bestandteile von ursprünglich murinen Antikörpern werden durch menschliche Teile ersetzt. Durch eine Humanisierung monoklonaler Antikörper der Maus können die bekannten Nachteile "klassischer" muriner Antikörper weitestgehend eliminiert werden, da der Körper diese nicht mehr oder weniger stark als "fremd" erkennt. Eine ähnliche Situation findet sich bei TCR (13, 14). Es besteht somit eine ständige Übung im Stand der Technik, murine Sequenzen durch menschliche zu ersetzen.

Ein alternatives Verfahren wurde in der DE 102 59 713.8 ("Verfahren zur Stabilisierung von Einzelketten-T-Zell-Rezeptoren") vorgestellt, bei dem die fehlende Calpha Domäne als trunkierte TCRalpha-Kette mit dem Einzelketten-TCR koexprimiert wird. Funktionalität ist ohne Fusion an ein signalgebendes Molekül gegeben. Dieses Verfahren ist jedoch auf murine Einzelketten-TCR beschränkt und konnte nicht auf einen humanen scTCR plus koexprimiertes humanes Calpha erweitert werden. Besonders bevorzugt ist laut DE 102 59 713.8 dabei eine scTCR-Kette oder scFv-Fragment, die/das humanisiert ist, was zu u.a. einer verbesserten Verträglichkeit des TCR oder Fragments führt.

Der vorliegenden Erfindung liegt im Hinblick auf das oben Gesagte die Aufgabe zugrunde, ein Verfahren zur Verfügung zu stellen, das die Herstellung eines Einzelketten-Antigen-erkennenden rekombinanten genetischen Konstrukts (scARC) mit einer verbesserten Expressionstabilisierung auch bei humanen scARC ermöglicht, so dass zum Beispiel bei der Herstellung rekombinanter scTCR nicht gemischte Pärchen mit den endogenen Ketten der T-Zellen ausgebildet werden. Zusätzlich zu der Stabilisierung der Zell-Oberflächenexpression des scTCR sollte eine messbare - und vor allem spezifische - Effektor-Funktion der humanen T-Zelle erreicht werden. Das Verfahren der Stabilisierung durch die geeignete Koexpression sollte sich zudem auch auf andere Einzelketten-Antigen-erkennende rekombinante genetische Konstrukte anwenden lassen, wie zum Beispiel scFv-Fragmente (2).

Die Aufgabe wird durch die Erfindung des Hauptanspruchs 1 und der nebengeordneten Ansprüche 9, 10, 11, 13, 14 und 15 gelöst. Die Unteransprüche beziehen sich auf spezielle Ausführungsformen der Erfindung.

Beschrieben wird ein Verfahren zur Herstellung einer ein stabilisiertes funktionelles Einzelketten-Antigen-erkennendes genetisches Konstrukt (scARC) exprimierenden Zellinie, umfassend a) zur Verfügung stellen einer geeigneten Wirtszelle, b) zur Verfügung stellen eines genetischen Konstrukts des zu exprimierenden humanen scARC, umfassend die Domänen huV_{1/2}-Li-huV_{2/1}-C_{4/3} und zur Verfügung stellen eines genetischen Konstrukts umfassend die entsprechende hetero-/(homo-)dimere Domäne C_{3/4}, enthaltend murine Aminosäureaustausche in die Domänen C_{4/3} und C_{3/4}, c) direktes oder indirektes Einbringen der genetischen Konstrukte über viralen oder nicht-viralen Gentransfer in eine rekombinante Zelle, und d) Koexpression der genetischen Konstrukte der scARC-Fragmente durch die Zelle.

Als Grund für das Versagen des Verfahrens wie beschrieben in der DE 102 59 713.8 wird vermutet, dass der kompetitive Druck zwischen der endogenen TCRalpha-Kette und des artifiziellen Calpha-Konstruktes um den Einbau in den essentiellen CD3-Komplex letztere weitgehend verdrängt. Humane native Doppelketten-TCR (dcTCR) bilden leichter hybride Paare mit endogenen TZR und dies verringert die Oberflächen-Expression des TAA-spezifischen Heterodimers, um so mehr wenn es sich dabei um eine trunkierte Calpha-Domäne handelt (14, 15). Die Murinisierung der konstanten Domäne in der trunkierten Calpha-Domäne sowie der Cbeta-Domäne im Einzelketten-TZR-Konstrukt bedingt eine präferentielle Interaktion der speziesfremden Ketten und dadurch dessen bevorzugten Einbau in den CD3-Komplex. Dieses unterstützt letztlich dessen Export zur Zellmembran.

Erfindungsgemäß wird daher durch Komponenten des murinen TCR der humane scTCR plus Calpha funktionell erhalten. Eine "Murinisierung" des humanen scTCR plus Calpha bedeutet, dass entsprechende Aminosäureaustausche, wie in der Maus-konstanten Domäne vorhanden, in die humanen konstanten Domänen des scTCR plus Calpha übertragen werden. Im Maximalfall werden die konstanten Domänen des scTCR plus Calpha durch murine Domänen ersetzt. Es werden allgemein so viele murine Austausche in die konstante Domäne eingeführt, wie für die effektive kompetitive Verdrängung der endogenen TCR-alpha Kette und dem Calpha-Konstrukt erforderlich sind. Der Fachmann ist ohne weiteres in der Lage, zu bestimmen und festzulegen, welche Zahl an Austauschen für den jeweiligen Fall effektiv ist. Diese Austausche finden sich in beiden rekombinanten Konstrukten, da diese die spezifische Paarung fördern sollen. Es ist möglich, auch xenogene, das heißt im Vergleich zur humanen Sequenz speziesfremde Punktmutationen einzuführen, die von den Maus-Sequenzen abweichen, aber einen vergleichbaren positiven Effekt auf die präferentielle Interaktion der TZR und deren Einbau in den CD3-Komplex bewerkstelligen.

Es ist auch vorstellbar, daß scARC zum Einsatz kommen, die humanen Ursprungs sind, aber durch z.B. Affinitätsmaturierung wie Phage Display (10) oder anderen Interventionen derart stark in den variablen, Antigen-erkennenden Domänen verändert sind, so daß diese nur noch als originär-humanen Ursprungs zu bezeichnen sind. Ebenso ist es denkbar, daß scARC aus anderen Spezies (allgemein Säuger) verwendet werden, die durch Sequenz-Veränderungen stark verändert sind und für den klinischen Einsatz geeignet sind. Der Begriff "xenogene" Aminosäureaustausche in den konstanten Domänen bezeichnet somit allgemein speziesspezifische Unterschiede zwischen variablen und konstanten Domänen.

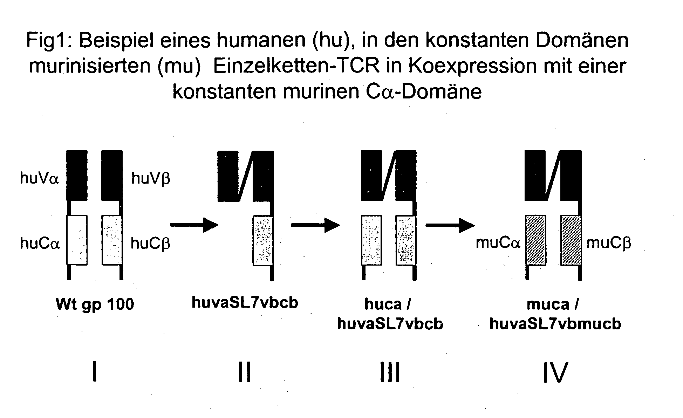

Da die konstante Domäne diejenige Domäne ist, die für die Membranverankerung und die Kopplung an CD3 zuständig ist, wurde diese Domäne modellhaft in einen als nativer TZR hoch-affinen humanen scTCR mit der gp100.280-288-Spezifität eingeführt und die murine Calpha Domäne koexprimiert (Fig. 1). Beide murine Domänen sind maßgeblich für Membranverankerung und CD3-Komplex-Integration (16) essentiell.

Der erfindungsgemäße Ansatz basiert somit auf einer Expressionsstabilisierung von Einzelketten-TCR (scTCR) durch die Koexpression der hetero-/(homo-)dimeren konstanten murinisierten Domäne als Bindungspartner und auf einer Expressionsstabilisierung von scFv-Fragmenten durch die Koexpression der hetero-/(homo-)dimeren murinisierten konstanten Domäne als Bindungspartner.

Bevorzugt ist ein Verfahren, das weiterhin die Präsentation des stabilisierten heterodimeren scARC durch die Zelle umfasst.

Ein weiterer bevorzugter Aspekt der vorliegenden Erfindung betrifft somit auch ein Verfahren zur Herstellung eines stabilisierten Einzelketten-TCR (scTCR). Das Verfahren umfaßt die Schritte wie in Anspruch 1 genannt und weiter die Aufreinigung des scARC aus der Zelle und, gegebenenfalls, die Rekonstitution der translatierten scARC-Fragmente in einer T-Zelle.

Es wurde erfindungsgemäß bevorzugt eine murinisierte Cα-Domäne konzipiert, die mit einem scTCR kotransferiert und somit koexprimiert werden kann. Hiermit sollte eine Stabilisierung der Zell-Oberflächenexpression des scTCR und eine meßbare Effektor-Funktion der humanen T-Zelle erreicht werden. Zusätzlich kann hierdurch eine unerwünschte Paarung mit endogenen Ketten verhindert werden. Nach dem Einschleusen der Kettenkombinationen in humane T-Zellen mussten diese in Hinblick auf ihre strukturelle Avidität, d.h. ihre strukturelle Integrität, als auch in Hinblick auf ihre funktionelle Avidität, d.h. das Aufrechterhalten der Peptidabhängigen Effektor-Funktion, getestet werden (17). Als Modellsystem dienten die humanen gp100(280-288)-spezifischen T-Zell-Rezeptoren. Die zusätzlich vorhandene, im scTCR jeweils fehlende konstante murinisierte Domäne, dient der Stabilisierung des Einzelketten-T-Zell-Rezeptors. Mit der Erhöhung der Stabilität ist eine Verbesserung der Expression und der Funktionalität im Kontext der T-Zell-Effektor-Funktion verbunden (dazu siehe Figuren).

Durch die stabilisierende Wirkung der zusätzlichen konstanten Domäne in Kombination mit einem scTCR ist es gelungen, funktionsfähige chimäre Rezeptoren ohne eine notwendige Fusion zur CD3ζ-Kette, zu entwickeln.

Die Kotransduktion mit (z.B.) Cα komplettiert die im Wildtyp-TCR vorhandenen vier extracytosolischen Domänen Cα, Cβ, Vα, Vβ und verändert ausschließlich die Verknüpfung dieser vier Domänen. Im cytosolischen und transmembranen Bereich als maßgebliche, Signalinduzierende Wirkorte ergibt sich somit kein struktureller Unterschied zum heterodimeren Wildtyp-TCR, ganz im Gegensatz zum TCR-CD3ζ-Konzept. Ein analoges Konzept besteht für den Fall der Komplettierung von konstanten Domänen bei scFv-Fragmenten. Zudem wird der Anteil der variablen Domänen der scARC, welcher der Antigenerkennung dient, durch die koexprimierte Domäne nicht sterisch benachteiligt.

Besonders bevorzugt ist ein Verfahren der vorliegenden Erfindung, wobei der scARC ein Einzelketten-TCR (scTCR) oder ein Antikörper scFv-Fragment ist.

Besonders bevorzugt ist ein Verfahren der vorliegenden Erfindung, wobei die Zelle eine Säuger-, insbesondere humane, T-Zelle ist. Weiter bevorzugt wird der scTCR in der Orientierung SP-Vα-Linker-Vβ-Cβ zusammen mit der konstanten Domäne SP-Cα oder der scARC in der Orientierung SP-Vβ-Linker-Vα-Cα zusammen mit der konstanten Domäne SP-Cβ koexprimiert. Diese Paarungen bilden dann optimale stabilisierte und richtig gepaarte scTCR aus. Das Signalpeptid (SP) wird normalerweise im Endoplasmatischen Retikulum (ER) abgespalten, kann aber in artifiziellen Fusionen zum Membranprotein auch weiterhin existent sein.

Neben dem MHC-restringierten scTCR-Konzept ermöglicht das oben genannte scFv-Konzept die MHC-unabhängige Erkennung von Antigenen: hierbei werden die Antigen-spezifischen variablen Domänen der schweren und leichten Kette eines Antikörpers mittels eines mindestens 14 Aminosäuren langen Linkers verknüpft und üblicherweise über eine "hinge"-Region, die als Abstandshalter zur Zellmembran dient, an membranständige, signalgebende Moleküle der CD3ζ-Kette oder der γ-Kette des Immunglobulin-Rezeptors FcR fusioniert. Als Abstandshalter fungieren die "hinge"-Region von CD8, die "hinge"-Region von IgG1 bzw. die Immunglobulin-ähnlichen Domänen C_{H2}C_{H3} von Antikörpern (7). Das scFv-Konzept impliziert, dass beliebige der oben erwähnten "hinge"-Regionen mit beliebigen der oben zitierten signalgebenden Komponenten chimärisiert werden können und diese mit beliebigen Antigen(Ag)-spezifischen Einzelketten-Fv-Fragmenten fusioniert werden können. Das hier beschriebene Konzept zur Stabilisierung und Funktionssteigerung von Einzelketten-TZR unter Wegfall der signalgebenden Komponente (CD3ζ) mittels der komplementären murinisierten konstanten Domäne ist auf das scFv-Konzept projizierbar: scFv-Fragmente werden z.B. an die murine konstante Cβ eines beliebigen TZR fusioniert und analog mit einer murinen Cα-Domäne kotransferiert. Auch ist es vorstellbar, die C_{H2}C_{H3}-Domänen eines Antikörpers zu verwenden, die auf der einen Seite mit dem scFv und auf der anderen Seite mit der membranständigen Domäne eines TCR chimärisiert wurden. Entsprechend wird eine Chimäre aus SP - C_{H2}C_{H3} - TM(TCR) - Cyt(TCR) hergestellt (Kürzel siehe Erklärung unten), die als komplementäre Domäne mit dem chimären scFv heterodimerisiert (18). Analog den natürlichen Gegebenheiten eines Antikörpers homodimerisieren die C_{H2}C_{H3}-Domänen unter Ausbildung eines bivalenten Antigen-spezifischen Moleküls. Sowohl C_{H2}C_{H3}-Domänen als auch Cα/Cβ-Domänen bilden untereinander stabilisierende Disulfidbrücken aus. Denkbar wäre auch die in den Fab-Fragmenten von Antikörpern liegende C_{L}-Domäne der leichten Kette mit der C_{H1}-Domäne der schweren Kette zu heterodimerisieren.

Diesen beschriebenen konstanten Domänen gemeinsam ist ihre Immunglobulin-ähnliche Faltung und ihre Neigung, sich durch Homo- bzw. Heterodimeriserung zu einer komplementären Domäne zu stabilisieren. Beispielhaft sind folgende TCR-abgeleitete wie auch Antikörperabgeleitete Chimäre vorstellbar: 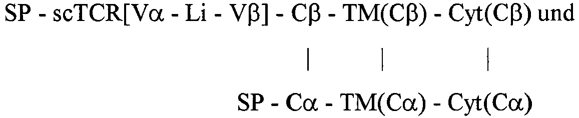 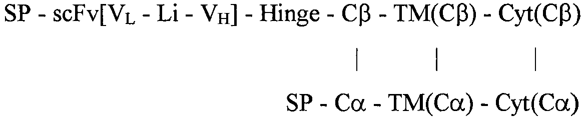 und
SP = Signalpeptid
V_{L} = F_{V}-Fragment der leichten Kette eines Antikörpers
V_{H} = F_{V}-Fragment der schweren Kette eines Antikörpers
Hinge = Verknüpfungsregion aus Antikörpern
TM(Cβ) = Transmembrane der murinisierten konstanten Domäne eines β-Ketten TCR
Cyt(Cβ) = Cytoplasmatische Anteil der murinisierten konstanten Domäne eines β-Ketten TCR
Die Beschreibung in den eckigen Klammern präzisiert das zuvor benannte Einzelketten(sc)-Konstrukt. Senkrechte Striche deuten Wechselwirkungen zwischen benachbarten Domänen an.

Die Integration einer aus Antikörpern entnommenen "hinge"-Region gilt nicht notwendig. Das in diesem Absatz beschriebene Konzept lässt die Bedeutung und Notwendigkeit der α-CPM-Domäne der α-Kette eines TCR im Fall der Verwendung von C_{H2}C_{H3} bzw. C_{H1} oder C_{L} unberücksichtigt. Vorstellbar wäre die Integration von C_{H2}C_{H3} bzw. C_{H1} oder C_{L} anstelle der in oben skizzierten Konstrukten verwendeten Cβ bzw. Cα. Die Anordnung der variablen Domänen in den Einzelketten (z.B. Vα-Li-Vβ versus Vβ-Li-Vα) ist beliebig und diktiert nicht notwendigerweise die Verwendung der sich direkt anschließenden konstanten Domäne (z.B. Vα versus Vβ).

Das "T-body"-genannte Konzept umfaßt alle chimären Doppelketten- und Einzelketten-Rezeptoren, die neben dem Membrananker als wesentliches Bestandteil die variablen Domänen Antigen-spezifischer Antikörper (Anti-"body") beinhalten (2) und z.B. durch viralen und/oder nicht-viralen Gentransfer in einen Thymozyt ("T"-cell) eingebracht werden.

Durch die Möglichkeit in dem hier vorgestellten, neuen Verfahren auf die signalgebende CD3ζ-Domäne am C-Terminus der scTCR- bzw. scFv-Chimären verzichten zu können, könnten funktionell andere signalgebende Molelüle wie die der zu der Syk-Familie gehörigen Tyrosinkinasen fusioniert werden (19). Hierdurch kann neben dem Erhalt der konformationellen Stabilität zusätzlich eine Optimierung der Signaltransduktion erreicht werden.

Ein weiterer Aspekt der vorliegenden Erfindung betrifft ein erfindungsgemäßes Verfahren, wobei der Linker (Li) ausgewählt ist aus Li(Gly₄Ser)₃, Li218 (20) und LiSL7 (21). Es können aber auch andere geeignete Linker verwendet werden, da das erfindungsgemäße Verfahren nicht auf die hier genannten Linker beschränkt ist.

Das erfindungsgemäße Verfahren kann für alle Einzelketten-Antigen-erkennenden Konstrukte verwendet werden. Als Antigene kommen alle durch diese Konstrukte erkannten Antigene in Frage, so zum Beispiel Erkrankungs-spezifische Oberflächen-Antigene, wie zum Beispiel TAA oder Infektions-spezifische Oberflächen-Antigene, wie zum Beispiel HIV-spezifische Oberflächen-Antigene oder CMV-spezifische Oberflächen-Antigene. Natürlich können auch weitere eingesetzt werden, die dem Fachmann bekannt sind.

Die Einbringung in die T-Zielzellen kann auf jede bekannte Weise erfolgen, die eine anschließende Expression des stabilisierten scTCR durch die T-Zelle ermöglicht. Wege sind zum Beispiel über die Induktion von Phagocytose durch die Zellen oder ein Verfahren, wobei die Einbringung durch Lipid-vermittelten Transfer, wie zum Beispiel über Micellen oder Liposomen-Transfer erfolgt. Eine Übersicht über die Verwendung von Liposomen gibt u.a. der Artikel Banerjee R. Liposomes: applications in medicine. J Biomater Appl 2001 Jul; 16(1):3-21. Der Transfer über Mizellen ist dem Fachmann im Stand der Technik aus zahlreichen Publikationen bekannt.

Dabei erfolgt somit eine Expression des z.B. scTCR außerhalb der zuletzt vorgesehenen exprimierenden T-Zelle und eine anschließende Einbringung des scTCR und der ebenfalls vorhandenen heterologen konstanten Domäne in dieselbe. Bei der *in vitro* Translation kann die Translation in zellfreien Systemen erfolgen, die käuflich erhältlich sind. Die "Translation" umfaßt aber auch die rein synthetische Herstellung der Peptidketten, die weiter unten im Rahmen der Peptide genauer erläutert wird. Im Falle der *in vivo* Translation kann diese in einer geeigneten Wirtszelle erfolgen, die vorher mit einem Expressionskonstrukt der Kette transformiert wurde und diese anschließend herstellt. Geeignete Vektoren und Verfahren zur Expression sind dem Fachmann im Stand der Technik ausreichend bekannt. Nach der Expression kann es erforderlich sein, die Expressionsprodukte entweder aus den Zellen zu reinigen oder aus dem Medium zu extrahieren, in das sie gegebenenfalls durch die Wirtszelle sekretiert wurden. Geeignete Wirtszellen sind ebenfalls bekannt und können Hefe, CHO-Zellen, Insektenzellen, Bakterien oder andere sein.

Gemäß einem weiteren Aspekt der Erfindung können beide scARC-Fragmente auf einem genetischen Konstrukt lokalisiert sein. Dadurch werden der scARC und die heterologe konstante Domäne in der richtigen Stöchiometrie von 1:1 exprimiert.

Bevorzugterweise wird erfindungsgemäß als scARC ein mit zusätzlichen (funktionellen) Domänen versehener scARC oder ein mit alternativen Domänen versehener scARC, z.B mit einer anderen Transmembran-Domäne als Membrananker versehener scARC, verwendet.

Bevorzugterweise wird erfindungsgemäß als scTCR ein alpha/beta scTCR, gamma/delta scTCR, einen mit zusätzlichen (funktionellen) Domänen versehenen scTCR oder einen mit alternativen Domänen versehenen scTCR, z.B mit einer anderen Transmembran-Domäne als Membrananker versehenen scTCR verwendet. Dabei können als alpha-Kette und beta-Kette die alpha- und beta-Ketten eines gp100(280-288)-spezifischen scTCR verwendet werden. Anhand dieses scTCR konnte das erfindungsgemäße Prinzip erstmals erfolgreich angewendet werden.

Bevorzugt ist weiterhin ein erfindungsgemäßes Verfahren, wobei als Transfektionssystem ein retroviraler Vektor, insbesondere pBullet verwendet wird. In den Vektoren können auch IRES-Elemente verwendet werden (22).

Ein weiterer Aspekt der vorliegenden Erfindung betrifft einen stabilisierten scARC, der nach einem Verfahren gemäß der vorliegenden Erfindung hergestellt wird. Weiter besonders bevorzugt ist ein erfindungsgemäß stabilisiertes scARC, der ein TAA-spezifischer scTCR oder ein TAA-spezifischer scFv-ARC ist, insbesondere ein mutierter gp100(280-288)-spezifischer TCR. Dieser stabilisierte scTCR gemäß der vorliegenden Erfindung kann auch in Form eines Fusionsproteins, umfassend die erfindungsgemäß modifizierten Polypeptide oder Teile davon vorliegen. Das Fusionsprotein kann dadurch gekennzeichnet sein, dass es die ζ-Region von CD3 oder CD8 oder CD16 oder Teile davon umfaßt, insbesondere die ζ-Region von humanem CD3 oder CD8 oder CD16 oder Teile davon. Insbesondere kann das erfindungsgemäße Fusionsprotein die ζ-Kette des CD3-Komplexes oder ITAM-Motive der ζ-Kette oder Teile davon umfassen, insbesondere die ζ-Kette von humanem CD3 oder Teile davon. Das Fusionsprotein kann weiterhin dadurch gekennzeichnet sein, dass es CD8α oder das Lck-Bindungsmotiv von CD8α umfaßt oder Teile davon, insbesondere von humanem CD8α.

Ein weiterer Aspekt der Erfindung betrifft eine isolierte Nukleinsäure, die eine für eine murinisierte konstante Domäne SP-Cα oder konstante murinisierte Domäne SP-Cβ1 oder murinisierte SP-Cβ2 kodierende Sequenz eines stabilisierten scTCR umfaßt. Diese erfindungsgemäße Nukleinsäure kann eine DNA, RNA, PNA (Peptide nucleic acid) oder p-NA (Pyranosyl nucleic acid), vorzugsweise eine DNA, insbesondere eine doppelsträngige DNA mit einer Länge von mindestens 8 Nukleotiden, vorzugsweise mit mindestens 18 Nukleotiden, insbesondere mit mindestens 24 Nukleotiden sein. Die Nukleinsäure kann dadurch gekennzeichnet sein, dass die Sequenz der Nukleinsäure mindestens ein Intron und/oder eine polyA-Sequenz aufweist. Sie kann auch in Form ihrer antisense-Sequenz vorliegen.

Ein weiterer Aspekt der Erfindung betrifft auch ein DNA- oder RNA-Vektormolekül, das mindestens eine oder mehrere erfindungsgemäße Nukleinsäure(n) umfaßt und das in Zellen exprimierbar ist. Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt mit dem ersten in einer Kozak Konsensus Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt. Eine weitere Verwendung der erfindungsgemäßen Nukleinsäuresequenzen ist die Konstruktion von anti-sense Oligonukleotiden (Zheng und Kemeny, 1995, Clin. Exp. Immunol. 100:380-2) und/oder Ribozymen (Amarzguioui, et al. 1998, Cell. Mol. Life Sci. 54:1175-202; Vaish, et al., 1998, Nucleic Acids Res. 26:5237-42; Persidis, 1997, Nat. Biotechnol. 15:921-2). Mit anti-sense Oligonukleotiden kann man die Stabilität der erfindungsgemäßen Nukleinsäure verringern und/oder die Translation der erfindungsgemäßen Nukleinsäure inhibieren. So kann beispielsweise die Expression der entsprechenden Gene in Zellen sowohl in vivo als auch in vitro verringert werden. Oligonukleotide können sich daher als Therapeutikum eignen. Diese Strategie eignet sich beispielsweise auch für Haut, epidermale und dermale Zellen, insbesondere, wenn die antisense Oligonukleotide mit Liposomen komplexiert werden (Smyth et al., 1997, J. Invest. Dermatol. 108:523-6; White et al., 1999, J. Invest. Dermatol. 112:699-705). Für die Verwendung als Sonde oder als "antisense" Oligonukleotid ist eine einzelsträngige DNA oder RNA bevorzugt.

Neben den aus Zellen isolierten natürliche Nukleinsäuren können alle erfindungsgemäßen Nukleinsäuren oder deren Teile auch synthetisch hergestellt worden sein. Weiterhin kann zur Durchführung der Erfindung eine Nukleinsäure verwendet werden, die synthetisch hergestellt worden ist. So kann die erfindungsgemäße Nukleinsäure beispielsweise chemisch anhand der beschriebenen Proteinsequenzen unter Heranziehen des genetischen Codes z. B. nach der Phosphotriester-Methode synthetisiert werden (siehe z. B. Uhlmann, E. & Peyman, A. (1990) Chemical Revievs, 90, 543-584).

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (WO 95/11910; Macadam et al., 1998, WO 98/37240; Reese et al., 1997, WO 97/29116). Typischerweise kann eine solche Stabilisierung durch die Einführung von einer oder mehrerer Internukleotid-Phosphorgruppen oder durch die Einführung einer oder mehrerer Nicht-Phosphor-Intemukleotide, erhalten werden.

Geeignete modifizierte Internukleotide sind in Uhlmann und Peymann (1990 Chem. Rev. 90, 544) zusammengefasst (WO 95/11910; Macadam et al., 1998, WO 98/37240; Reese et al., 1997, WO 97/29116). Modifizierte Internukleotid-Phosphatreste und/oder Nicht-Phosphorbrücken in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat, Phophatester, während Nicht-Phosphor-Internukleotid-Analoge, beispielsweise Siloxanbrücken, Carbonatbrücken, Carboxymethylester, Acetamidatbrücken und/oder Thioetherbrücken enthalten. Es ist auch beabsichtigt, daß diese Modifizierung die Haltbarkeit einer pharmazeutischen Zusammensetzung, die bei einer der erfindungsgemäßen Verwendungen eingesetzt werden kann, verbessert.

Ein weiterer Aspekt der vorliegenden Erfindung betrifft einen Vektor, vorzugsweise in Form eines Plasmids, shuttle Vektors, Phagemids, Cosmids, Expressionsvektors, adenoviralen Vektors, retroviralen Vektors (Miller, et al. "Improved retroviral vectors for gene transfer and expression", BioTechniques Vol. 7, No. 9, p 980, 1989) und/oder gentherapeutisch wirksamen Vektors, der eine erfindungsgemäße Nukleinsäure enthält.

So kann die erfindungsgemäße Nukleinsäure in einem Vektor vorzugsweise in einem Expressionsvektor oder gentherapeutisch wirksamen Vektor enthalten sein. Vorzugsweise enthält der gentherapeutisch wirksame Vektor T-Zell-spezifische regulatorische Sequenzen, die funktionell mit der erfindungsgemäßen Nukleinsäure verbunden sind. Die Expressionsvektoren können prokaryotische oder eukaryotische Expressionsvektoren sein. Beispiele für prokaryotische Expressionsvektoren sind für die Expression in *E. coli* z.B. die Vektoren pGEM oder pUC-Derivate und für eukaryotische Expressionsvektoren für die Expression in *Saccharomyces cerevisiae* z. B. die Vektoren p426Met25 oder p426GAL1 (Mumberg et al. (1994) Nucl. Acids Res., 22, 5767-5768), für die Expression in Insektenzellen z. B. *Baculovirus*-Vektoren wie in EP-B1-0 127 839 oder EP-B1-0 549 721 offenbart, und für die Expression in Säugerzellen z. B. die Vektoren Rc/CMV und Rc/RSV oder SV40-Vektoren, welche alle allgemein erhältlich sind.

Im allgemeinen enthalten die Expressionsvektoren auch für die jeweilige Wirtszelle geeignete Promotoren, wie z. B. den trp-Promotor für die Expression in E. coli (siehe z. B. EP-B1-0 154 133), den Met 25, GAL 1 oder ADH2-Promotor für die Expression in Hefen (Russel et al. (1983), J. Biol. Chem. 258, 2674-2682; Mumberg, supra), den Baculovirus-Polyhedrin-Promotor für die Expression in Insektenzellen (siehe z. 13. EP-B1-0 127 839). Für die Expression in Säugetierzellen sind beispielsweise Promotoren geeignet, die eine konstitutive, regulierbare, gewebsspezifische, zellzyklusspezifische oder metabolischspezifische Expression in eukaryotischen Zellen erlauben. Regulierbare Elemente gemäß der vorliegenden Erfindung sind Promotoren, Aktivatorsequenzen, Enhancer, Silencer und/oder Repressorsequenzen. Beispiel für geeignete regulierbare Elemente, die konstitutive Expression in Eukaryonten ermöglichen, sind Promotoren, die von der RNA Polymerase III erkannt werden oder virale Promotoren, CMV-Enhancer, CMV-Promotor, CMV-LTR-Hybride, SV40 Promotor oder LTR-Promotoren z. B. von MMTV (mouse mammary tumour virus; Lee et al. (1981) Nature 214, 228-232) und weitere virale Promotor- und Aktivatorsequenzen, abgeleitet aus beispielsweise HBV, HCV, HSV, HPV, EBV, HTLV oder HIV. Ein Beispiel für ein regulierbares Element, das eine regulierbare Expression in Eukaryonten ermöglicht, ist der Tetracyclinoperator in Kombination mit einem entsprechenden Repressor (Gossen M. et al. (1994) Curr. Opin. Biotechnol. 5, 516-20).

Beispiele für regulierbare Elemente, die T-Zell spezifische Expression in Eukaryonten ermöglichen, sind Promotoren oder Aktivatorsequenzen aus Promotoren oder Enhancern von solchen Genen, die für Proteine kodieren, die nur in diesen Zelltypen exprimiert werden.

Beispiele für regulierbare Elemente, die Zellzyklus-spezifische Expression in Eukaryonten ermöglichen, sind Promotoren folgender Gene: cdc25, Cyclin A, Cyclin E, cdc2, E2F, B-myb oder DHFR (Zwicker J. und Müller R. (1997) Trends Genet. 13, 3-6). Beispiele für regulierbare Elemente, die metabolischspezifische Expression in Eukaryonten ermöglichen, sind Promotoren, die durch Hypoxie, durch Glukosemangel, durch Phosphatkonzentration oder durch Hitzeschock reguliert werden.

Der erfindungsgemäße Vektor kann zur Transfektion einer Wirtszelle verwendet werden, bei der es sich bevorzugterweise um eine T-Zelle handelt. Besonders bevorzugt ist eine Wirtszelle, die dadurch gekennzeichnet ist, dass sie auf ihrer Oberfläche einen erfindungsgemäß stabilisierten scTCR oder Fusionsprotein oder stabilisiertes scFv-Fragment exprimiert. Ein weiterer Gegenstand der Erfindung betrifft somit ein Verfahren zur Herstellung eines Polypeptids zur Diagnose und/oder Behandlung von mit Onkoproteinen in Zusammenhang stehenden Erkrankungen oder zur Identifizierung von pharmakologisch aktiven Substanzen in einer geeigneten Wirtszelle, das dadurch gekennzeichnet ist, dass eine erfindungsgemäße Nukleinsäure auf geeignete Weise exprimiert wird.

Das Polypeptid wird so beispielsweise durch Expression der erfindungsgemäßen Nukleinsäure in einem geeigneten Expressionssystem, wie oben bereits beschrieben, nach dem Fachmann allgemein bekannten Methoden hergestellt. Als Wirtszellen eignen sich beispielsweise die *E. coli* Stämme DHS, HB101 oder BL21, der Hefestamm *Saccharomyces cerevisiae,* Insektenzellinien, z. B. von *Spodoptera frugiperda,* oder die tierischen Zellen COS, Vero, 293, HaCaT, und HeLa, die alle allgemein erhältlich sind.

Um die Einführung von erfindungsgemäßen Nukleinsäuren und damit die Expression des Polypeptids in einer eu- oder prokaryotischen Zelle durch Transfektion, Transduktion, Transformation oder Infektion zu ermöglichen, kann die Nukleinsäure als Plasmid, als Teil eines viralen oder nicht-viralen Vektors oder Partikels vorliegen. Als virale Vektoren oder Partikel eignen sich hierbei besonders: Baculoviren, Vakziniaviren, Retroviren, Adenoviren, adenoassoziierte Viren und Herpesviren. Als nicht-virale Träger eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren oder retrovirale Vektoren (Lindemann et al., 1997, Mol. Med. 3: 466-76; Springer et al., 1998, Mol. Cell. 2: 549-58; (4)
Ein bevorzugter Mechanismus, erfindungsgemäße Polypeptide *in vivo* zur Expression zu bringen, ist der virale Gen-Transfer, insbesondere mit Hilfe retroviraler Partikel. Diese werden vorzugsweise genutzt, entsprechende Zielzellen, vorzugsweise T-Lymphozyten, des Patienten *ex vivo* mit den für erfindungsgemäße Polypeptide kodierenden Genen oder Nukleotidsequenzen durch Transduktion zu versehen. Die Zielzellen können daraufhin im Sinne eines adoptiven Zelltransfers wieder in den Patienten reinfundiert werden, um mit der *de novo* eingefügten Spezifität tumorizide und/oder immunmodulierende Effektorfunktionen zu übernehmen. Jüngst wurden auf diesem Wege sehr gute gentherapeutische Erfolge in der Behandlung der durch Immuninkompetenz gekennzeichneten SCID-X1-Krankheit bei Neugeborenen erzielt, in dem hämatologische Vorläuferzellen mit einem analogen intakten Transgen einer in den Kindern vorkommenden nicht-funktionellen mutierten Variante des γ-Kettengens, das für die Differenzierung in die verschiedenen Effektorzellen des adaptiven Immunsystems essentiell ist, retroviral versehen wurden (23).

Weiterhin besteht die Möglichkeit den Gentransfer *in vivo* durchzuführen, einerseits durch präferentiell stereotaktische Injektion der infektiösen Partikel, andererseits durch direkte Applikation von Viren-produzierenden Zellen (Oldfield, et al. Hum. Gen. Ther., 1993, 4:39-69).

Die zum Transfer von Genen häufig eingesetzten viralen Vektoren sind nach heutigem Stand der Technik vorwiegend retrovirale, lentivirale, adenovirale und adeno-assoziierte virale Vektoren. Diese sind von natürlichen Viren abgeleitete zirkuläre Nukleotidsequenzen, in denen zumindest die viralen Strukturprotein-kodierenden Gene durch das zu transferierende Konstrukt ausgetauscht werden.

Retrovirale Vektorsysteme schaffen die Voraussetzung für eine langanhaltende Expression des Transgens durch die stabile, aber ungerichtete Integration in das Wirtsgenom. Vektoren der jüngeren Generation besitzen keine irrelevanten und potentiell immunogenen Proteine, des weiteren gibt es keine vorbestehende Immunität des Empfängers gegenüber dem Vektor. Retroviren enthalten ein RNA-Genom, das in eine Lipidhülle verpackt ist, die aus Teilen der Wirtszellmembran und Virusproteinen besteht. Zur Expression viraler Gene wird das RNS-Genom revers transkribiert und mit dem Enzym Integrase in die Zielzell-DNS integriert. Diese kann daraufhin von der infizierten Zelle transkribiert und translatiert werden, wodurch virale Bestandteile entstehen, die sich zu Retroviren zusammenfügen. RNS wird ausschließlich dann in die neu entstandenen Viren eingefügt. Das Genom der Retroviren besitzt drei essentielle Gene: *gag*, das für virale Strukturproteine, so genannte gruppenspezifische Antigene kodiert, *pol* für Enzyme wie Reverse Transkriptase und Integrase und *env* für das Hüllprotein ("envelope"), das für die Bindung des wirtsspezifischen Rezeptors verantwortlich ist. Die Produktion der Replikations-inkompetenten Viren findet nach Transfektion in so genannten Verpackungszelllinien statt, die zusätzlich mit den gag/pol-kodierenden Genen ausgestattet wurden und diese "in trans" exprimieren und somit die Ausbildung Replikationsinkompetenter (d.h. gag/pol-deletierter) transgener Viruspartikel komplementieren. Eine Alternative ist die Cotransfektion der essentiellen Virusgene, wobei nur der das Transgen enthaltende Vektor das Verpackungssignal trägt.

Die Separation dieser Gene ermöglicht einerseits die beliebige Kombination des gal/pol-Leserahmens mit aus verschiedenen Stämmen gewonnenen env-Leserahmen, wodurch Pseudotypen mit verändertem Wirtstropismus entstehen, andererseits kann dadurch die Bildung replikationskompetenter Viren innerhalb von Verpackungszellen drastisch reduziert werden. Das aus "gibbon ape leukemia virus" (GALV) abgeleitete Hüllprotein, das im "stitch" bzw. "bullet"-Vektorsystem Verwendung findet (4, 7), ist in der Lage humane Zellen zu transduzieren und ist in der Verpackungszelllinie PG13 mit amphotropen Wirtsbereich etabliert. Zusätzlich wird die Sicherheit durch selektive Deletion von nicht-essentiellen Virussequenzen zur Verhinderung einer homologen Rekombination und somit der Produktion replikationskompetenter Partikel erhöht.

Neue, nicht-virale Vektoren bestehen aus autonomen, sich selbst-integrierenden DNS-Sequenzen, den Transposonen, die durch z.B. liposomale Transfektion in die Wirtszelle eingeschleust werden und erstmals erfolgreich zur Expression humaner Transgene in Säugerzellen eingesetzt wurden (24, 25).

Gentherapeutisch wirksame Vektoren lassen sich auch dadurch erhalten, daß man die erfindungsgemäße Nukleinsäure mit Liposomen komplexiert, da damit eine sehr hohe Transfektionseffizienz, insbesondere von Hautzellen, erreicht werden kann (Alexander und Akhurst, 1995, Hum. Mol. Genet. 4: 2279-85). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6, 282; Brandén et al. (1999) Nature Biotech. 17, 784). Hilfsstoffe umfassen auch Moleküle, die die Freisetzung von Nukleinsäuren in das Cytoplasma der Zelle ermöglichen (Planck et al. (1994) J. Biol. Chem. 269, 12918; Kichler et al. (1997) Bioconj. Chem. 8, 213) oder beispielsweise Liposomen (Uhlmann und Peymann (1990) supra). Eine andere besonders geeignete Form von gentherapeutischen Vektoren läßt sich dadurch erhalten, daß man die erfindungsgemäße Nukleinsäure auf Goldpartikeln aufbringt und diese mit Hilfe der sogenannten "Gene Gun" in Gewebe, bevorzugt in die Haut, oder Zellen schießt (Wang et al., 1999, J. Invest. Dermatol., 112:775-81). Für die gentherapeutische Anwendung der erfindungsgemäßen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Jackson, R. J. (1993) Cell 74, 9-14 und Palmiter, R. D. et al. (1991) Proc. Natl. Acad. Sci. USA 88, 478-482).

Die vorliegende Beschreibung richtet sich auch auf eine Wirtszelle, die ein DNA- oder RNA-Vektormolekül gemäß der Erfindung enthält. Diese kann insbesondere eine T-Zelle sein, die mit einem erfindungsgemäßen Vektor oder einem anderen erfindungsgemäßen Genkonstrukt transformiert ist. Wirtszellen können sowohl prokaryontische als auch eukaryontische Zellen sein, Beispiele für prokaryontische Wirtszellen sind *E. coli* und für eukaryontische Zellen *Saccharomyces cerevisiae* oder Insektenzellen.

Beschrieben wird somit eine rekombinante T-Zelle, die mindestens einen stabilisierten scTCR gemäß der vorliegenden Erfindung exprimiert. Eine transformierte Wirtszelle ist eine transgene T-Vorläuferzelle oder eine Stammzelle, die dadurch gekennzeichnet ist, dass sie ein erfindungsgemäßes Genkonstrukt oder eine erfindungsgemäße Expressionskassette umfaßt. Verfahren zur Transformation oder Transduktion von Wirtszellen und/oder Stammzellen sind dem Fachmann gut bekannt und umfassen zum Beispiel Elektroporation oder Mikroinjektion. Eine besonders bevorzugte transformierte Wirtszelle ist eine patienteneigene T-Zelle, die nach der Entnahme mit einem erfindungsgemäßen Genkonstrukt transfiziert wird. Erfindungsgemäße Wirtszellen können insbesondere dadurch erhalten werden, dass dem Patienten eine oder mehrere Zellen, bevorzugterweise T-Zellen, insbesondere CD8⁺-T-Zellen entnommen werden, die dann *ex vivo* mit einem oder mehreren erfindungsgemäßen genetischen Konstrukten transfiziert oder transduziert werden, um so erfindungsgemäße Wirtszellen zu erhalten. Die *ex vivo* generierten spezifischen T-Zellen können dann anschließend in den Patienten reimplantiert werden. Das Verfahren ähnelt somit dem bei Darcy et al. ("Redirected perforin-dependent lysis of colon carcinoma by ex vivo genetically engineered CTL" J. Immunol., 2000, 164:3705-3712) beschriebenen Verfahren unter der Verwendung von scFv anti-CEA Rezeptor transduzierten ZTL, Perforin und γ-IFN.

Die erfindungsgemäß modifizierten (Poly)peptide und deren Derivate können zum Beispiel auch zur aktiven und/oder passiven Immunisierung von Patienten mit Erkrankungen, insbesondere Tumorerkrankungen, die zum Beispiel mit gp100 in Zusammenhang stehen, eingesetzt werden. Die Beschreibung betrifft somit eine Verwendung bei der eine Krebserkrankung behandelt wird, insbesondere eine Krebserkrankung, die mit einer veränderten Expression von gp100 in Zusammenhang steht, um so die Induktion, Erzeugung und Zunahme vom Onkogen-spezifischen, z.B. gp100-spezifischen CTL zu erreichen und die Tumor- und Leukämiezellen der betreffenden Patienten spezifisch abzutöten. Solche Erkrankungen umfassen zum Beispiel solide Tumorerkrankungen, lymphohämatopoetische Neoplasien, maligne hämatologische Erkrankungen, auch in Form eines multiplen Myeloms (oder Plastozytoms), eines histiozytischen Lymphoms und eines CML-Blastenschubs. Damit zusamenhängende TAAs, gegen die entsprechende TCR entwickelt werden können, sind zum Beispiel p53, Her-2/neu, Ras, Tyrosinase, MART, Gp100, MAGE, BAGE, MUC-1, TRP-1, TRP-2, CD45, CD19 und PRDI-BF1.

Ein besonders bevorzugter Aspekt der vorliegenden Erfindung betrifft somit auch eine Zusammensetzung, insbesondere pharmazeutische Zusammensetzung, die eine rekombinante T-Zelle gemäß der vorliegenden Erfindung umfaßt. Bevorzugt ist weiterhin die Verwendung eines stabilisierten scTCR gemäß der vorliegenden Erfindung, eines mutierten TCR gemäß der vorliegenden Erfindung und/oder einer rekombinanten T-Zelle gemäß der vorliegenden Erfindung zur Herstellung von Therapeutika und/oder Prophylaktika zur Behandlung von Krebserkrankungen. Bei einer besonders bevorzugten Art der Behandlung wird dem Patienten eine oder mehrere Zellen, bevorzugterweise T-Zellen, insbesondere CD8⁺-T-Zellen entnommen, die dann ex vivo mit einem oder mehreren erfindungsgemäßen genetischen Konstrukten transduziert oder transfiziert werden. Die *ex vivo* generierten spezifischen T-Zellen können dann anschließend in den Patienten re-implantiert werden. Die erfindungsgemäße Zusammensetzung kann weiterhin geeignete Zusatz- und Hilfsstoffe enthalten.

Ein Gegenstand der vorliegenden Beschreibung ist auch ein Arzneimittel zur Indikation und Therapie von mit Onkoprotein-Protein assoziierten Erkrankungen, das eine erfindungsgemäße Nukleinsäure oder ein erfindungsgemäßes Polypeptid und gegebenenfalls geeignete Zusatz- oder Hilfsstoffe enthält, sowie ein Verfahren zur Herstellung eines solchen Arzneimittels zur Behandlung von mit Onkoprotein-Protein assoziierten Erkrankungen, bei dem eine erfindungsgemäße Nukleinsäure oder ein erfindungsgemäßes Polypeptid mit einem pharmazeutisch annehmbaren Träger formuliert wird. Als Therapeutika und/oder Prophylaktika kommen insbesondere Impfstoffe, rekombinante Partikel oder Injektionen oder Infusionslösungen in Betracht, die als Wirkstoff (a) das erfindungsgemäße stabilisierte scTCR-Rezeptor Polypeptid und/oder seine Derivate und/oder (b) eine erfindungsgemäße Nukleinsäure enthalten und/oder (c) *in vitro* oder *ex vivo* erzeugte T-Lymphozyten, die einen spezifisch mutierten gegen Onkoprotein gerichteten scTCR enthalten.

Für die gentherapeutische Anwendung beim Menschen ist vor allem ein Arzneimittel und/oder rekombinanter Partikel geeignet, das die erfindungsgemäße Nukleinsäure in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8. Insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z. B. Nukleaseinhibitoren, vorzugsweise Komplexbildner wie EDTA und/oder andere dem Fachmann bekannte Hilfs-stoffe enthalten.

Es ist denkbar, die Effizienz des Cα/scTCR-Konzeptes weiter durch Chimärisierung an signalgebende Domänen wie CD3ζ oder Tyrosinkinasen zu verbessern (z.B. Cα-ζ/scTCR-ζ). Verschiedene scTCR-Konstrukte, unabhängig ob humanen oder murinen Ursprungs sowie der Natur des Linkers und der gewählten Orientierung der variablen Domänen, werden in diesem Ansatz mitberücksichtigt.

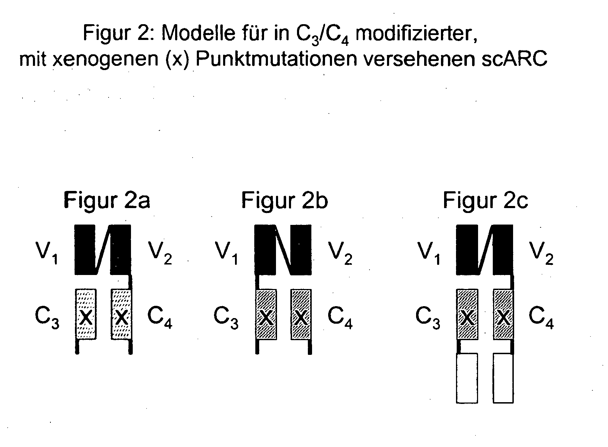

CD3ζ-Ketten besitzen neben ihrer signalgebenden Funktion, das Potential zur Homodimerisierung und erleichtern somit die Assoziation einer chimären Cαζ-Kette mit dem chimären scTCRζ (25). Neben dem hier als Beispiel beschriebenen Ansatz eines murinisierten humanen TZR der Orientierung SP-V₁-Li-V₂-C₄ plus eine koexprimierte trunkierte C₃-Domäne (Figur 2a) besteht eine weitere Möglichkeit darin, inverse Konstrukte der Orientierung SP-V₂-Li-V₁-C₃ plus C₄ zu konzipieren, welche im Fall eines TZR als Beispiel anstelle der Cβ-Domäne eine Cα-Domäne beinhalten und in Kombination mit einer SP-Cβ-Domäne kotransferiert werden (Figur 2b). Ferner ist es denkbar, beliebige variable Domänen, von Antikörpern oder T-Zell-Rezeptoren abgeleitet, an beliebige invariante Domänen in ihrer Funktion als a) Abstandshalter zur Transmembranen, b) als Dimerisierungsdomäne, c) als Membrananker und d) als Adapterprotein zur Signalkopplung zu fusionieren (Figur 2c). Das vorgestellte Konzept ist zudem Spezies-unabhängig und kann auf humane Moleküle übertragen werden. Es können zur weiteren Verallgemeinerung xenogene Punktmutationen, im Sonderfall ganze Domänen anderer Spezies (z.B. Ratte, Affe, Hund), in C₃ und C₄ eingeführt werden, die einen stabilisierenden Effekt auf die Avidität des Einzelketten-Konstruktes bewirken.

Zudem ist es denkbar, dass das scTCR stabilisierende konstante murinisierte Domänen-Konzept auf Einzelketten-Antikörper (scFv) durch die Koexpression der komplementären CH_{L}- bzw C_{L}-Kette auszudehnen. Das Konzept ist somit generalisiert.

Alle bisher und künftig entwickelten TAA-spezifischen TCR, die in der adoptiven Immuntherapie durch Gentransfer in humane T-Zellen von Tumorpatienten verwendet werden sollen, können mit geringem Aufwand nach beschriebenem Modell entwickelt werden. Der vorgestellte Ansatz könnte somit eine weite Verbreitung in der klinischen Applikation finden (23).

Der Begriff "stabilisierter scARC" bezieht sich auf ein stabilisiertes Einzelkettenantigenerkennendes genetisches Konstrukt (scARC), bei dem zusätzlich zu dem herkömmlichen V_{1/2}-Li-V_{2/1}-C_{4/3}-sc-Konstrukt eine entsprechende heterodimere konstante punktmutierte oder xenogene, im Sonderfall murinisierte Domäne C_{3/4} koexprimiert wird. Bevorzugterweise handelt es sich bei den scARC um stabilisierte Einzelketten-TCR (scTCR) oder stabilisierte Antikörper-scFv-Fragmente, die weiter bevorzugt tumorassoziierte Peptidantigene (TAA) erkennen. Der Begriff "stabilisierter scTCR" bezieht sich auf die Kombination des jeweiligen scTCR, z.B. in der Orientierung SP-Vα-Li-Vβ-Cβ zusammen mit der konstanten Domäne SP-Cα oder des scTCR in der Orientierung SP-Vβ-Li-Vα-Cα zusammen mit der konstanten Domäne SP-Cβ. Diese Paarungen bilden dann optimale stabilisierte und richtig gepaarte scTCR aus. Dabei können die stabilisierten scTCR aus der Expression eines oder zweier verschiedener genetischer Konstrukte stammen.

Der Begriff "kodierende Nukleinsäure" bezieht sich auf eine DNA-Sequenz, die für ein isolierbares bioaktives erfindungsgemäßes Polypeptid oder einen Vorläufer kodiert. Das Polypeptid kann durch eine Sequenz in voller Länge oder jeden Teil der kodierenden Sequenz kodiert werden, solange die spezifische, beispielsweise enzymatische Aktivität erhalten bleibt.

Es ist bekannt, dass kleine Veränderungen in der Sequenz der erfindungsgemäßen Nukleinsäuren vorhanden sein können, zum Beispiel durch die Degenerierung des genetischen Codes, oder daß nicht translatierte Sequenzen am 5' und/oder 3'-Ende der Nukleinsäure angehängt sein können, ohne dass dessen Aktivität wesentlich verändert wird. Diese Erfindung umfaßt deshalb auch sogenannte "funktionelle Varianten" der erfindungsgemäßen Nukleinsäuren.

Unter "stringenten Hybridisierungsbedingungen" sind solche Bedingungen zu verstehen, bei denen eine Hybridisierung bei 60°C in 2,5 x SSC-Puffer, gefolgt von mehreren Waschschritten bei 37°C in einer geringeren Pufferkonzentration erfolgt und stabil bleibt.

Die Erfindung soll nun weiter anhand der beigefügten Beispiele und Figuren erläutert werden, die insoweit sie über den Gegenstand der Ansprüche hinaus gehen, Vergleichsbeispiele darstellen. Es zeigt:
SEQ ID Nr. 1: die Nukleinsäuresequenz des in Cbeta murinisierten Einzelketten T-Zell-Rezeptors der gp100.280-288-Spezifität von Hu Chim scTCR gp100 (scHuVαLiHuVβ-MuCβ),
SEQ ID Nr. 2: die Nukleinsäuresequenz eines murinen trunkierten TCRalpha mit Signalpeptid aus TCRalpha von (Mu SpCα),
SEQ ID Nr. 3: die Proteinsequenz von Hu Chim scTCR gp100 (scHuVαLiHuVβMuCβ), und
SEQ ID Nr. 4: die Proteinsequenz von Mu SpCα,
wobei hu = human, mu = murin, Li = Linker, SP = Signalpeptid ist.
Figur 1: Beispiel eines humanen (hu), in den konstanten Domänen murinisierten (mu) Einzelketten-TCR in Koexpression mit einer konstanten murinen Ca-Domäne
   In Figur 1 bezeichnet I die Struktur eines nativen humanen (hu) TCR, bestehend aus zwei heterodimeren alpha- und beta-Ketten. Jede Kette ist aus 2 Domänen aufgebaut, von denen die variablen Domänen (Vα/Vβ) für die Antigen-Erkennung und die konstanten Domänen (Cα/Cβ) für die Membranverankerung und Kopplung an den signalgebenden CD3-Komplex verantwortlich sind. II bezeichnet ein Einzelketten-TZR-Konstrukt, bei dem die variablen Domänen über ein 19 Aminosäure-langen Glycin/Serin-reichen Linker verknüpft sind. Die Calpha-Domäne fehlt; die Membranverankerung erfolgt ausschließlich über Cbeta. III bezeichnet ein Einzelketten-TZR-Konstrukt, bei dem die humane Calpha-Domäne unabhängig koexprimiert wird. IV bezeichnet ein chimäres Einzelketten-TZR-Konstrukt, bei dem die humane Cbeta-Domäne gegen eine murine Cbeta-Domäne ersetzt wurde. Aus Gründen der Komplementarität wird dieses mit einer ebenso murinen Calpha-Domäne koexprimiert.
Figur 2: Modelle für in C3/C4 modifizierter, mit xenogenen (x) Punktmutationen versehenen scARC
Figur 2a/b bezeichnet die denkbaren Kombinationen aus humanen variablen Domänen (V₁ und V₂) und ganz oder teilweise mit xenogenen (X) Aminosäureaustauschen ausgestatteten konstanten Domänen (C₁ und C₂). Figur 2c illustriert die mögliche Chimärisierung an funktionell andere, z.B. signalgebende Domänen.
Figur 3: Oberflächenexpression humaner gp100.280-288 spezifischer TZR-Konstrukte (FACS-Analytik)

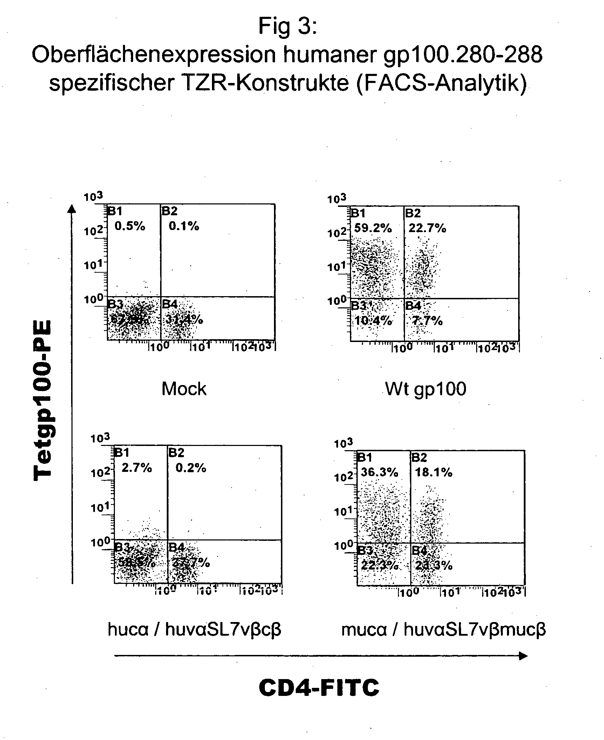

   Figur 3 zeigt die Analyse der Oberflächenexpression durch FACS. Mit Hilfe eines CD4-FITC-Antikörpers wird in CD4+ und CD8+ T-Zellen unterschieden. Alle Zellen sind CD3+ und daher T-Zellen. Das Tetramer besteht aus multimeren HLA-A2-Molekülen, die mit dem gp100.280-288 - Peptid beladen sind. Konstrukte mit intakter struktureller Avidität sind in der Lage an diese zu binden. Mock bezeichnet die leeren retroviralen Vektoren, die übrigen Konstrukte sind der Figur 1 zu entnehmen. Um eine maximal denkbare Expression zu gewährleisten, sind alle Transduktionsansätze auf Resistenz gegen die Antibiotika Neomycin und Puromycin selektiert worden. Die Koexpression des humanen Calpha bedingt keinen Nachweis, erst die Murinisierung beider konstanten Domänen zeitigt eine nachweisbare Oberflächenexpression.

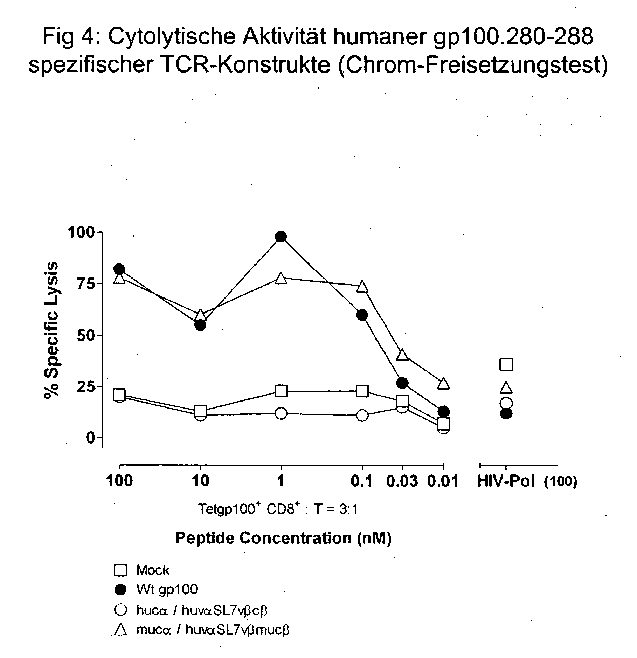

Figur 4: Cytolytische Aktivität humaner gp100.280-288 spezifischer TCR-Konstrukte (Chrom-Freisetzungstest)
   Figur 4 zeigt die Analyse der funktionellen Avidität mit Hilfe des radioaktiven Chrom-Freisetzungstest. Konstrukte wie in der Figur 1 bezeichnet, sind darauf getestet worden, T2-Zielzellen mit dem relevanten gp100-sezifischen Antigen dosisabhängig beladen, gegenüber einem irrelevanten Antigen spezifisch zu erkennen. Rekombinante CD8+ T-Zellen lysieren via Perforin je nach Effizienz des jeweiligen Konstruktes die erkannten Zielzellen. Das murinisierte Konstrukt IV der Figur 1 ist in seiner lytischen Effizienz dem nativen TCR vergleichbar trotz geringerer Expression (Figur 2).

### Beispiele

Hinsichtlich der Herstellung und entsprechender molekularbiologischer Methoden kann grundsätzlich auf die Beispiele der DE 102 59 713.8 verwiesen werden.

Im Rahmen der Versuche für die vorliegende Erfindung wurde durch Komponenten des murinen TCR der humane scTCR plus Calpha funktionell erhalten. Da die konstante Domäne diejenige Domäne ist, die für die Membranverankerung und die Kopplung an CD3 zuständig ist, wurde diese Domäne modellhaft in einen als nativer TZR hoch-affinen humanen scTCR mit der gp100.280-288-Spezifität eingeführt und die murine Calpha Domäne koexprimiert (Fig 1). Beide murine Domänen sind maßgeblich für Membranverankerung und CD3-Komplex-Integration essentiell.

Im Gegensatz zum human scTCR plus humanes Calpha, das keinerlei Oberflächenexpression im FACS zeigte, war die des chimären scTCR plus murines Calpha deutlich meßbar (Fig. 2.: 36.3 % der CD8+ und 18.1 % der CD4+ T-Zellen). Ebenso wurde eine über den Hintergrund deutlich meßbare cytolytische Aktivität im Chrom-Freisetzungstest gemessen (Fig. 3). Diese war den wildtypischen dcTCR vergleichbar, obwohl die Expression eingeschränkt zu sein scheint. Es wurde durch die Steigerung der Expression hoch-affiner dcTCR keine weitere Verbesserung der cytolytischen Aktivität erwartet.

Dieses Beispiel zeigt an Hand des gp 100.280-288-spezifischen TCR die Möglichkeit, humane Einzelketten-TZR durch partielle Murinisierung in humanen T-Zellen funktionell zu initiieren ohne den Chimärismus des Moleküls zu sehr zu erhöhen, das heißt es zu sehr zu verfremden und damit die Antigenizität sowie deren unspezifische Reaktivität zu erhöhen.

In weiterführenden Versuchen wurde gezeigt, daß bei einem erfindungsgemäßen Konstrukt umfassend eine Ecto-Subdomäne mit einer murinen Sequenz und einem Transmembran- und Cytosolteil mit einer humanen Sequenz eine Aktivität gefunden wurde, die zwar nicht so hoch war, wie bei einer vollständig murinisierten konstanten Domäne gemäß der Erfindung (siehe oben), jedoch immer noch deutlich ausgeprägt war, wohingegen bei einem Konstrukt umfassend eine Ecto-Subdomäne mit einer humanen Sequenz und einem Transmembran- und Cytosolteil mit einer murinen Sequenz eine Aktivität gefunden wurde, die fast gegen null geht. Dieses Experimente zeigte somit klar die Bedeutung der Murinisierung der Ecto-Subdomäne.

### Versuchsanleitungen:

### Transduktion humaner peripherer Blut Lymphozyten (PBLs)

Zur Transduktion humaner peripherer T-Lymphozyten wurde ein funktionelles Derivat des pStitch-Systems (Weijtens et al., 1999) verwendet. Die zur Verpackung notwendigen retroviralen Gene werden über individuelle Plasmide im Wege einer Cotransfektion der Verpakkungszellinie 293T kodiert: pHit60 kodiert für die *gag-pol -* Struktur- und Polymerase - Gene aus dem Moloney murinen Leukämie Virus (MoMuLV), pColt-Galv für das *env -* Hüllprotein des "gibbon ape leukemia virus", das imstande ist, an den Na⁺-/Phosphat-Synporter Pit humaner Zellen zu binden und damit letztere zu transduzieren. Die chimären Viruspartikel besitzen somit einen amphotropen Pseudotyp und können verschiedene Säugerzellen, außer Maus, transduzieren.

### Transfektion der Verpackungszelllinie 293T

Die isolierten bakteriellen Klone der in das pStitch-Derivat klonierten T-Zell-Rezeptor-Gene wurden über Plasmid-Präparationen, die eine Entfernung residueller Endotoxine versprechen (Qiagen, Produkt 12362), gereinigt und zu 0.5 µg/µl eingestellt. Die DNS wurde über die Calciumphosphat-Präzipitation transient in die Verpackungszelllinie 293T eingeführt (GiboBRL-Life Technologies, Produkt 18306-019). Hierbei werden im Rahmen der stabilisierten T-Zell-Rezeptoren αTCR und βTCR bis zu 80 µg DNS eingesetzt:
20 µg αTCR - Konstrukt
20 µg βTCR - Konstrukt
20 µg pColt-Galv
20 µg pHit 60

Im Fall von Einzelketten-TCRs werden 60 µg DNS eingesetzt. 293T wurde in einem modifizierten DMEM-Medium (DMEM/H) kultiviert:
DMEM, 4,5% Glucose (BioWhittaker)
10% hitzeinaktiviertes FKS
2mM Glutamin
1 x Penicillin/Streptomycin
1x nicht-essentielle Aminosäuren
25 mM HEPES

Am Vortag der Transfektion wurden die Zellen 293T auf 0,9*10⁶ Zellen pro T25-Flasche und Transfektionsansatz in 5ml DMEM/H ausgesät. 4 h vor Transfektion wurde das Medium gegen frisches, auf Raumtemperatur (RT) erwärmtes DMEM-H (3ml) ersetzt. Die Transfektion erfolgte laut Vorschrift des kommerziellen Protokolls (Invitrogen). 1 ml des Transfektionsansatzes wurde zu der jeweiligen Flasche unter vorsichtigem Hinzutropfen pipettiert. Das DNS-Ca₃(PO₄)₂-Präzipität sollte fein verteilt sich auf den adhärenten Zellen niederlegen.

Am darauf folgenden Morgen wurde das Medium gegen frisches, auf RT erwärmtes DMEM/H ausgetauscht. 6 h später erfolgte die Kokultivierung mit den aktivierten PBLs.

### Transduktion der aktivierten PBLs - Aktivierung peripherer Blut-Lymphozyten (PBLs)

3 Tage vor der angesetzten Kokultivierung wurden fikollisierte PBLs zu 2*10⁶ Zellen/ml in huRPMI-P jeweils in 2 ml einer 24 Well-Platte (Zellgewebe-behandelte Oberflächen) ausgesät. Die Aktivierung erfolgte über den kreuz-vemetzenden Antikörper OKT-3 (Orthoclone-Diagnostics) zu 20 ng/ml.
huRPMI-P:
   RPMI 1640 (2mM Glutamin) ohne Phosphat (Life Tec., 11877-032)
   10 % humanes, hitzeinaktiviertes AB-Serum (HLA-A2.1 seropositiv)
   25 mMHEPES
   1 x Penicillin/Streptomycin (Life Tec.)
Die Platten wurden in einem Brutschrank bei 37°C und 5% CO2 inkubiert.

### Kokultivierung

Zur Kokultivierung wurden die aktivierten PBLs aus den jeweiligen Vertiefungen einer 24-Well-Platte vereinigt und gezählt. Adhärente Monozyten wurden verworfen. Die Zellen werden abzentrifugiert (1500 U/min, 5 min, RT) und in einer Konzentration von 2,5*10⁶ Zellen/ml in frischem huRPMI-P aufgenommen und in den Brutschrank zurückgestellt. Das Medium wurde zuvor zu 400 U/ml IL-2 (Chiron) und 5 µg/ml Polybren (Sigma) eingestellt.

Jeder Transfektionsansatz wurde 6h nach Mediumwechsel nacheinander trypsiniert: hierzu wurde jede T25 mit 3 ml HBSS (Life Technologies) gewaschen, mit 1 ml Trypsin-EDTA (Life Technologies) für maximal 5 Minuten inkubiert, die gelösten Zellen quantitativ aufgenommen und in 4ml vorgelegtes huRPMI-P (RT) unter Rühren getropft. Die 293T Zellen werden mit 2500 Rad bestrahlt. Diese werden abzentrifugiert (1500 U/min, 5 min, RT) und in 4 ml frisches, eingestelltes huRPMI-P, mit 400U/ml IL-2 und 5µg/ml Polybren supplementiert, resuspendiert. Zu dem Ansatz wurde 1 ml der eingestellten PBLs gegeben und der Ansatz (0,5*10⁶ PBLs/ml) für drei Tage im Brutschrank (37°C, 5% CO₂) inkubiert.

Am Tag 3 nach Kokultivierung wurden die suspendierten PBLs abgenommen und in frischem, mit 40 U/ml IL-2 (Chiron) und 2,5 µl CD3/CD28-Beads supplementiertem Medium huRPMI-P zu 1*10⁶ Zellen/ml resuspendiert. 3 Tage später erfolgte ein neuerlicher Split in frisches Medium. In diesen 7 Tagen wurde maximal expandiert bis zum Übergang auf T75-Flaschen. Diese Zellen konnten direkt in einer immunologischen Anfärbung (FACS-Analyse) oder in einem klassischen ⁵¹Chrom-Freisetzungstest eingesetzt werden.

### Beispiele der FACS-Analyse

Die oben beschriebenen Konstrukte wurden nach retroviraler Transduktion im "fluorescence activated cell sorting" (FACS) analysiert. Hierzu wurden 0,25*10⁶ Zellen sättigend mit Fluorophor-markierten Antikörpern gefärbt: der heterolog exprimierte TZR wurde mit einem anti-gp100.280-288-spezifischem Tetramer, einem Multimer aus peptidbeladenen HLA-A2.1-Molekülen, nachgewiesen; die Verteilung der T-Zellen zu CD4⁺/CD8⁺ über den Marker anti-CD4-FITC (Coulter-Beckman). Als Negativ-Kontrolle diente eine mit dem leeren pStitch-Derivat transduzierte Probe (mock). Die Expression konnte in mehreren Donoren HLA-A2 - positiver T-Zellen reproduziert werden. Die Tetramere wurden im Labor von Herrn Prof. P. Romero (Universität Lausanne, Schweiz) synthetisiert (26).

### Cytolytische Aktivität der transduzierten T-Zellen

Die transduzierten T-Zellen wurden in einem klassischen ⁵¹Chrom-Freisetzungstest auf ihre zytotoxische Spezifität hin überprüft. In diesem System wurden Zielzellen radioaktiv durch Inkorporation von ⁵¹Chrom markiert. Erkannten die retroviral modifizierten Effektorzellen die Zielzelle peptidspezifisch, wurden letztere durch die Effektorfunktionen der T-Zelle in die Apoptose getrieben und durch Lyse getötet. Das Ausmaß des freigesetzten Chrom-Nuklids trifft eine Aussage über die Effektivität der Zell-Erkennung und Lyse. Die Effektivität wurde über einen weiten Bereich des Verhältnisses eingesetzter Effektor-Zellen zu Zielzellen (E:T) getestet und zu Tetgp110⁺CD8⁺:T korrigiert. Als Referenz diente der wildtypische gp100.280-288 spezifische Doppelketten-TZR, aus dem chimären Einzelketten-TZR angefertigt wurden. Als Zielzelle diente:
T2: humane TAP-defiziente Zelllinie die exogen mit beliebigem Peptid zu beladen war. Das spezifische mutierte Peptid war MDM2-81-88, ein irrelevantes Kontroll-Peptid entstammte der HIV-Polymerase (AS 510-518).

### Zitierte Literatur

1. Schumacher, T. N. (2002) Nat. Rev. Immunol. 2, 512-519.
2. Eshhar, Z., Waks, T., Gross, G. & Schindler, D. G. (1993) Proc. Natl. Acad. Sci. U. S. A 90, 720-724.
3. Chung, S., Wucherpfennig, K. W., Friedman, S. M., Hafler, D. A. & Strominger, J. L. (1994) Proc. Natl. Acad. Sci. U. S. A 91, 12654-12658.
4. Willemsen, R. A., Weijtens, M. E., Ronteltap, C., Eshhar, Z., Gratama, J. W., Chames, P. & Bolhuis, R. L. (2000) Gene Ther. 7, 1369-1377.
5. Willcox, B. E., Gao, G. F., Wyer, J. R., O'Callaghan, C. A., Boulter, J. M., Jones, E. Y., van der Merwe, P. A., Bell, J. I. & Jakobsen, B. K. (1999) Protein Sci. 8, 2418-2423.
6. Mosquera, L. A., Card, K. F., Price-Schiavi, S. A., Belmont, H. J., Liu, B., Builes, J., Zhu, X., Chavaillaz, P. A., Lee, H. I., Jiao, J. A. et al. (2005) J. Immunol. 174, 4381-4388.
7. Weijtens, M. E., Willemsen, R. A., van Krimpen, B. A. & Bolhuis, R. L. (1998) Int. J. Cancer 77, 181-187.
8. Hombach, A., Heuser, C., Gerken, M., Fischer, B., Lewalter, K., Diehl, V., Pohl, C. & Abken, H. (2000) Gene Ther. 7, 1067-1075.
9. Zhang, T., He, X., Tsang, T. C. & Harris, D. T. (2004) Cancer Gene Ther. 11, 487-496.
10. Li, Y., Moysey, R., Molloy, P. E., Vuidepot, A. L., Mahon, T., Baston, E., Dunn, S., Liddy, N., Jacob, J., Jakobsen, B. K. et al. (2005) Nat. Biotechnol. 23, 349-354.
11. Tartaglia, J., Bonnet, M. C., Berinstein, N., Barber, B., Klein, M. & Moingeon, P. (2001) Vaccine 19, 2571-2575.
12. Kalergis, A. M., Boucheron, N., Doucey, M. A., Palmieri, E., Goyarts, E. C., Vegh, Z., Luescher, I. F. & Nathenson, S. G. (2001) Nat. Immunol. 2, 229-234.
13. Voss, R. H., Kuball, J. & Theobald, M. (2005) Methods Mol. Med. 109, 229-256.
14. Voss, R. H., Kuball, J., Engel, R., Guillaume, P., Romero, P., Huber, C. & Theobald, M. (2006) Immunol. Res. 34,67-87.
15. Rubinstein, M. P., Kadima, A. N., Salem, M. L., Nguyen, C. L., Gillanders, W. E., Nishimura, M. I. & Cole, D. J. (2003) J. Immunol. 170, 1209-1217.
16. Call, M. E., Pyrdol, J., Wiedmann, M. & Wucherpfennig, K. W. (2002) Cell 111, 967-979.
17. Bullock, T. N., Mullins, D. W., Colella, T. A. & Engelhard, V. H. (2001) J. Immunol. 167, 5824-5831.
18. Roberts, M. R., Qin, L., Zhang, D., Smith, D. H., Tran, A. C., Dull, T. J., Groopman, J. E., Capon, D. J., Byrn, R. A. & Finer, M. H. (1994) Blood 84, 2878-2889.
19. Fitzer-Attas, C. J., Schindler, D. G., Waks, T. & Eshhar, Z. (1998) J. Immunol. 160, 145-154.
20. Whitlow, M., Bell, B. A., Feng, S. L., Filpula, D., Hardman, K. D., Hubert, S. L., Rollence, M. L., Wood, J. F., Schott, M. E., Milenic, D. E. et al. (1993) Protein Eng 6, 989-995.
21. Robinson, C. R. & Sauer, R. T. (1998) Proc. Natl. Acad. Sci. U. S. A 95, 5929-5934.
22. Rees, S., Coote, J., Stables, J., Goodson, S., Harris, S. & Lee, M. G. (1996) Biotechniques 20, 102-110.
23. Cavazzana-Calvo, M., Hacein-Bey, S., de Saint, B. G., Gross, F., Yvon, E., Nusbaum, P., Selz, F., Hue, C., Certain, S., Casanova, J. L. et al. (2000) Science 288, 669-672.
24. Yant, S. R., Meuse, L., Chiu, W., Ivics, Z., Izsvak, Z. & Kay, M. A. (2000) Nat. Genet. 25, 35-41.
25. Bolliger, L. & Johansson, B. (1999) J. Immunol. 163, 3867-3876.
26. Robert, B., Guillaume, P., Luescher, I., Romero, P. & Mach, J. P. (2000) Eur. J. Immunol. 30, 3165-3170.

## Patentansprüche

1. Verfahren zur Herstellung einer humanen Zellinie, die einen stabilisierten funktionellen humanen (hu) Einzelketten-TCR (scTCR) oder ein stabilisiertes humanes Antikörper Einzelketten Fv-Fragment (scFv) exprimiert, umfassend
a) zur Verfügung stellen einer geeigneten humanen Wirtszelle, die keine embryonale Stammzelle ist,
b) zur Verfügung stellen eines genetischen Konstrukts des zu exprimierenden humanen scTCR oder scFv-Fragments, umfassend die Domänen huVα-Linker-huVβ-Cβ oder huVβ-Linker-huVα-Cα des zu exprimierenden humanen scTCR, oder umfassend die Domänen huV_{L}-Linker-huV_{H}-Cβ oder huV_{H}-Linker-huV_{L}-Cα des zu exprimierenden humanen scFv; und
zur Verfügung stellen eines genetischen Konstrukts umfassend die jeweils entsprechende heterodimere Domäne Cα oder Cβ,
wobei der Linker eine beliebige Peptidsequenz darstellt und wobei die konstanten Domänen Cα oder Cβ ganz oder teilweise murinisiert sind,
c) direktes oder indirektes Einbringen der genetischen Konstrukte über Gentransfer in eine rekombinante Zelle, und
d) Koexpression der genetischen Konstrukte der scTCR- oder scFv-Fragmente durch die Zelle.

2. Verfahren nach Anspruch 1, wobei die humane Wirtszelle eine T-Zelle ist.

3. Verfahren nach einem der Ansprüche 1 oder 2, weiter umfassend e) die Präsentation des stabilisierten heterodimeren scTCR oder scFv durch die Zelle.

4. Verfahren nach einem der Ansprüche 1 bis 3, wobei der scTCR oder scFv in der Orientierung Signalpeptid (SP)-Vα-Linker-Vβ-Cβ, oder (SP)-V_{L}-Linker-V_{H}-Cβ, zusammen mit der konstanten Domäne SP-Cα oder der scTCR oder scFv in der Orientierung SP-Vβ-Linker-Vα-Ca, oder (SP)-V_{H}-Linker-V_{L}-Cα, zusammen mit der konstanten Domäne SP-Cβ koexprimiert wird.

5. Verfahren nach einem der Ansprüche 1 bis 4, wobei beide scTCR- oder beide scFV-Fragmente auf einem genetischen Konstrukt lokalisiert sind.

6. Verfahren nach einem der Ansprüche 1 bis 5, wobei es sich bei dem scTCR oder scFv um einen alpha/beta scTCR oder scFv, gamma/delta scTCR oder scFv oder um einen mit einer anderen Transmembran-Domäne als Membrananker versehenen scTCR oder scFv handelt.

7. Verfahren nach einem der Ansprüche 1 bis 6, weiter umfassend die Aufreinigung des scTCR oder scFV aus der Zelle.

8. Verfahren nach Anspruch 7, weiter umfassend die Rekonstitution der translatierten scTCR- oder scFv-Fragmente in einer T-Zelle.

9. Stabilisierter humaner Tumor assoziiertes Peptidantigen-spezifischer scTCR oder scFv, umfassend die Domänen huVα-Linker-huVβ-Cβ oder huVβ-Linker-huVα-Cα des scTCR, oder umfassend die Domänen huV_{L}-Linker-huV_{H}-Cβ oder huV_{H}-Linker-huV_{L}-Cα des scFv; und die entsprechende heterodimere Domäne Cα oder Cβ, wobei der Linker eine beliebige Peptidsequenz darstellt, und wobei die konstanten Domänen Cα oder Cβ ganz oder teilweise murinisiert sind.

10. Rekombinante humane Zellinie, die keine embryonale Stammzelllinie ist, umfassend ein genetisches Konstrukt eines zu exprimierenden humanen scTCR oder scFv, umfassend die Domänen huVα-Linker-huVβ-Cβ oder huVβ-Linker-huVα-Cα des zu exprimierenden humanen scTCR, oder umfassend die Domänen huV_{L}-Linker-huV_{H}-Cβ oder huV_{H}-Linker-huV_{L}-Cα des zu exprimierenden humanen scFv, und weiter umfassend ein genetisches Konstrukt umfassend die entsprechende heterodimere Domäne Cα oder Cβ, wobei der Linker eine beliebige Peptidsequenz darstellt, und wobei die konstanten Domänen Cα oder Cβ ganz oder teilweise murinisiert sind.

11. Isolierte Nukleinsäure, die eine für eine konstante Domäne Cα oder eine konstante Domäne Cβ kodierende Sequenz und eine für einen stabilisierten scTCR oder scFv nach Anspruch 9 kodierende Sequenz umfasst.

12. Isolierte Nukleinsäure nach Anspruch 11, die eine Sequenz umfasst, die für eine konstante Domäne Cα gemäß SEQ ID Nr. 2 oder für eine konstante Domäne Cβ gemäß SEQ ID Nr. 1 kodiert.

13. DNA- oder RNA-Vektormolekül, das mindestens eine oder mehrere Nukleinsäure(n) nach Anspruch 11 oder 12 umfasst und das in Zellen exprimierbar ist.

14. Pharmazeutische Zusammensetzung, die eine rekombinante Zellinie nach Anspruch 10 umfaßt.

15. Verwendung einer rekombinanten Zellinie nach Anspruch 10 oder eines stabilisierten scTCR oder scFv nach Anspruch 9 zur Herstellung von Therapeutika und/oder Prophylaktika zur Behandlung von Krebserkrankungen.

16. Verwendung nach Anspruch 15, wobei eine Krebserkrankung behandelt wird, die mit einer veränderten Expression von MDM2, p53, Her-2/neu, Ras, Tyrosinase, MART, Gp100, MAGE, BAGE, MUC-1, TRP-1, TRP-2, CD45, CD19 oder PRDI-BF1 in Zusammenhang steht.

## Claims

1. A method for producing a human cell line expressing a stabilized functional human single chain-TCR (scTCR) or a stabilized human antibody single chain Fv-fragment (scFv), comprising
a) providing of a suitable human host cell, which is not an embryonic stem cell,
b) providing of a genetic construct of the human scTCR or scFv-fragment to be expressed, comprising the domains huVα-linker-huVβ-Cβ or huVβ-linker-huVα-Cα of the human scTCR to be expressed, or comprising the domains huV_{L}-linker-huV_{H}-Cβ or huV_{H}-linker-huV_{L}-Cα of the human scFv;
and
providing of a genetic construct comprising the respectively corresponding hetero dimeric domain Cα or Cβ,
wherein the linker represents an arbitrary peptide sequence and wherein the constant domains Cα or Cβ are fully or partially murinized,
c) direct or indirect introduction of the genetic constructs via gene transfer into a recombinant cell, and
d) co-expression of the genetic constructs of the scTCR or scFv-fragments by the cell.

2. The method according to claim 1, wherein the human host cell is a T-cell.

3. The method according to claim 1 or 2, further comprising the presentation of the stabilized heterodimeric scTCR or scFv by the cell.

4. The method according to any of claims 1 to 3, wherein said scTCR or scFv is co-expressed in the orientation signal peptide (SP)-Vα-linker-Vβ-Cβ or (SP)-V_{L}-linker-V_{H}-Cβ together with the constant domain SP-Cα, or the scTCR or scFv is co-expressed in the orientation (SP)-Vβ-linker-Vα-Cα or (SP)-V_{H}-linker-V_{L}-Cα together with the constant domain SP-Cβ.

5. The method according to any of claims 1 to 4, wherein both scTCR- or both scFv-fragments are localised on one genetic construct.

6. The method according to any of claims 1 to 5, wherein said scTCR or scFv is an alpha/beta scTCR or scFv, gamma/delta scTCR or scFv, or is a scTCR or scFv provided with a different transmembrane-domain as membrane anchor.

7. The method according to any of claims 1 to 6, further comprising the purification of the scTCR or scFV from the cell

8. The method according to any one of claims 1 to 7, further comprising the reconstitution of the translated scTCR- or scFv-fragments in a T-cell.

9. Stabilized human tumor associated peptide antigen-specific scTCR or scFv, comprising the domains huVα-linker-huVβ-Cβ or huVβ-linker-huVα-Cα of the scTCR, or comprising the domains huV_{L}-linker-huV_{H}-Cβ or huV_{H}-linker-huV_{L}-Cα of the scFv, and the corresponding hetero dimeric domain Cα or Cβ, wherein the linker represents an arbitrary peptide sequence and wherein the constant domains Cα or Cβ are fully or partially murinized.

10. Recombinant human cell line, which is not an embryonic stem cell line, comprising a genetic construct of a human scTCR or scFv to be expressed, comprising the domains huVα-linker-huVβ-Cβ or huVβ-linker-huVα-Cα of the human scTCR to be expressed, or comprising the domains huV_{L}-linker-huV_{H}-Cβ or huV_{H}-linker-huV_{L}-Cα of the human scFv to be expressed, and further comprising a genetic construct comprising the corresponding hetero dimeric domain Cα or Cβ, wherein the linker represents an arbitrary peptide sequence and wherein the constant domains Cα or Cβ are fully or partially murinized.

11. Isolated nucleic acid comprising a sequence encoding for a constant domain Cα or a constant domain Cβ,, and a sequence encoding for a stabilized scTCR or scFv according to claim 9.

12. Isolated nucleic acid according to claim 11, comprising a sequence encoding for a constant domain Cα according to SEQ ID NO: 2 or a constant domain Cβ according to SEQ ID NO: 1.

13. DNA or RNA-vector molecule that comprises at least one or more nucleic acid(s) according to claims 11 or 12 and that can be expressed in cells.

14. Pharmaceutical composition comprising a recombinant cell line according to claim 10.

15. Use of a recombinant cell according to claim 10 or of a stabilized scTCR or scFv according to claim 9 for producing therapeutics and/or prophylactics for a treatment of cancerous diseases.

16. Use according to claim 15, whereby a cancerous disease is treated that is related to a modified expression of MDM2, p53, Her-2/neu, Ras, tyrosinase, MART, Gp100, MAGE, BAGE, MUC-1, TRP-1, TRP-2, CD45, CD19 or PRDI-BF1.

## Revendications

1. Procédé de préparation d'une lignée cellulaire humaine qui exprime un récepteur des cellules T (TCR) à chaîne unique (scTCR) humain (hu) fonctionnel stabilisé ou un fragment Fv à chaîne unique (scFv) d'anticorps humain stabilisé, comprenant les étapes suivantes consistant à :
a) fournir une cellule hôte humaine appropriée, qui n'est pas une cellule souche embryonnaire,
b) fournir une construction génétique du scTCR ou du fragment scFv humain à exprimer, comprenant les domaines huVα-séquence de liaison-huVβ-Cβ ou huVβ-séquence de liaison-huVα-Cα du scTCR humain à exprimer, ou comprenant les domaines huV_{L}-séquence de liaison-huV_{H}-Cβ ou huV_{H}-séquence de liaison-huV_{L}-Cα du scFv humain à exprimer ; et
fournir une construction génétique comprenant les domaines Cα ou Cβ hétérodimères correspondants respectifs,
la séquence de liaison représentant une séquence peptidique quelconque et les domaines constants Cα ou Cβ étant complètement ou partiellement murinisés,
c) introduire de manière directe ou indirecte les constructions génétiques par transfert génique dans une cellule recombinante, et
d) laisser la cellule co-exprimer les constructions génétiques des fragments scTCR ou scFv.

2. Procédé selon la revendication 1, dans lequel la cellule hôte humaine est une cellule T.

3. Procédé selon la revendication 1 ou 2, comprenant en outre l'étape consistant à :
e) laisser la cellule présenter l'hétérodimère scTCR ou scFv stabilisé.

4. Procédé selon l'une quelconque des revendications 1 à 3, dans lequel le scTCR ou le scFv est co-exprimé avec le domaine constant SP-Cα dans l'orientation peptide signal (SP)-Vα-séquence de liaison-Vβ-Cβ, ou (SP)-V_{L}-séquence de liaison-V_{H}-Cβ, ou le scTCR ou le scFv est co-exprimé avec le domaine constant SP-Cβ dans l'orientation SP-Vβ-séquence de liaison-Vα-Cα, ou (SP)-V_{H}-séquence de liaison-V_{L}-Cα.

5. Procédé selon l'une quelconque des revendications 1 à 4, dans lequel les deux fragments scTCR ou les deux fragments scFV sont localisés sur une construction génétique.

6. Procédé selon l'une quelconque des revendications 1 à 5, dans lequel le scTCR ou le scFv est un scTCR ou un scFv alpha/bêta, un scTCR ou un scFv gamma/delta ou un scTCR ou un scFv muni d'un autre domaine transmembranaire faisant office d'ancre membranaire.

7. Procédé selon l'une quelconque des revendications 1 à 6, comprenant en outre la récupération du scTCR ou du scFV à partir de la cellule.

8. Procédé selon la revendication 7, comprenant en outre la reconstitution des fragments scTCR ou scFv traduits dans une cellule T.

9. scTCR ou scFV stabilisé spécifique d'un antigène peptidique et associé à une tumeur humaine, comprenant les domaines huVα-séquence de liaison-huVβ-Cβ ou huVβ-séquence de liaison-huVa-Ca du scTCR, ou comprenant les domaines huV_{L}-séquence de liaison-huV_{H}-Cβ ou huV_{H}-séquence de liaison-huV_{L}-Cα du scFv; et les domaines Cα ou Cβ hétérodimères correspondants, la séquence de liaison représentant une séquence peptidique quelconque, et le domaine constant Cα ou Cβ étant complètement ou partiellement murinisés.

10. Lignée cellulaire humaine recombinante, qui n'est pas une lignée de cellules souches embryonnaires, comprenant une construction génétique d'un scTCR ou d'un scFV humain à exprimer, comprenant les domaines huVα-séquence de liaison-huVβ-Cβ ou huVβ-séquence de liaison-huVα-Cα du scTCR humain à exprimer, ou comprenant les domaines huV_{L}-séquence de liaison-huV_{H}-Cβ ou huV_{H}-séquence de liaison-huV_{L}-Cα du scFV humain à exprimer, et comprenant en outre une construction génétique comprenant les domaines Cα ou Cβ hétérodimères correspondants, la séquence de liaison représentant une séquence peptidique quelconque, et les domaines constants Cα ou Cβ étant complètement ou partiellement murinisés.

11. Acide nucléique isolé, qui comprend une séquence codant pour un domaine constant Cα ou un domaine constant Cβ et une séquence codant pour un scTCR ou un scFv stabilisé selon la revendication 9.

12. Acide nucléique isolé selon la revendication 11, qui comprend une séquence qui code pour un domaine constant Cα selon SEQ ID N° 2 ou pour un domaine constant Cβ selon SEQ ID N° 1.

13. Molécule vecteur d'ADN ou d'ARN, qui comprend au moins un ou plusieurs acide(s) nucléique(s) selon la revendication 11 ou 12 et qui peut être exprimée dans des cellules.

14. Composition pharmaceutique qui comprend une lignée cellulaire recombinante selon la revendication 10.

15. Utilisation d'une lignée cellulaire recombinante selon la revendication 10 ou d'un scTCR ou d'un scFv stabilisé selon la revendication 9 pour la préparation de produits thérapeutiques et/ou prophylactiques destinés au traitement de cancers.

16. Utilisation selon la revendication 15, dans laquelle est traité un cancer en lien avec une expression modifiée de MDM2, p53, Her-2/neu, Ras, une tyrosinase, MART, Gp100, MAGE, BAGE, MUC-1, TRP-1, TRP-2, CD45, CD 19 ou PRDI-BF1.


### SEQUENCE LISTING

<110> Johannes-Gutenberg-Universität Mainz
<120> Verfahren zur Verbesserung der spezifischen Effektorfunktion von Einzelketten-Antigen-erkennenden genetischen Konstrukten (scARC) durch deren Murinisierung
<130> FB21265
<160> 4
<170> PatentIn version 3.3
<210> 1
   <211> 1308
   <212> DNA
   <213> artificial
<220>
   <223> mutated scTCR construct (Valpha-Linker-Vbeta-Cbeta)
<400> 1
   atggcatcca ttcgagctgt atttatattc ctgtggctgc agctggactt ggtgaatgga      60
   gagaatgtgg agcagcatcc ttcaaccctg agtgtccagg agggagacag cgctgttatc     120
   aagtgtactt attcagacag tgcctcaaac tacttccctt ggtataagca agaacttgga     180
   aaaagacctc agcttattat agacattcgt tcaaatgtgg gcgaaaagaa agaccaacga     240
   attgctgtta cattgaacaa gacagccaaa catttctccc tgcacatcac agagacccaa     300
   cctgaagact cggctgtcta cttctgtgca gcaagtactt cgggtggtac tagctatgga     360
   aagctgacat ttggacaagg gaccatcttg actgtccacg gcaccagcgg cagcagtggt     420
   agcggcagcg gtggcagcgg tagtggcggt ggcggccaag tgacccagaa cccaagatac     480
   ctcatcacag tgactggaaa gaagttaaca gtgacttgtt ctcagaatat gaaccatgag     540
   tatatgtcct ggtatcgaca agacccaggg ctgggcttaa ggcagatcta ctattcaatg     600
   aatgttgagg tgactgataa gggagatgtt cctgaagggt acaaagtctc tcgaaaagag     660
   aagaggaatt tccccctgat cctggagtcg cccagcccca accagacctc tctgtacttc     720
   tgtgccagca gtttggggag ctcctacgag cagtacttcg ggccgggcac caggctcacg     780
   gttttagagg atctgagaaa tgtgactcca cccaaggtct ccttgtttga gccatcaaaa     840
   gcagagattg caaacaaaca aaaggctacc ctcgtgtgct tggccagggg cttcttccct     900
   gaccacgtgg agctgagctg gtgggtgaat ggcaaggagg tccacagtgg ggtcagcacg     960
   gaccctcagg cctacaagga gagcaattat agctactgcc tgagcagccg cctgagggtc    1020
   tctgctacct tctggcacaa tcctcgaaac cacttccgct gccaagtgca gttccacggg    1080
   ctttcagagg aggacaagtg gccagagggc tcacccaaac ctgtcacaca gaacatcagt    1140
   gcagaggcct ggggccgagc agactgtgga atcacttcag catcctatca tcagggggtt    1200
   ctgtctgcaa ccatcctcta tgagatccta ctggggaagg ccaccctata tgctgtgctg    1260
   gtcagtggcc tggtgctgat ggctatggtc aagaaaaaaa attcctga                 1308
<210> 2
   <211> 471
   <212> DNA
   <213> artificial
<220>
   <223> mutated TCRalpha construct (Signal peptide-Calpha)
<400> 2
   atggaaaggc tgctgtgctc tctgctgggg cttctgtgca cacaggtttg ctgggtgaaa      60
   ggacagaacc cagaacctgc tgtgtaccag ttaaaagatc ctcggtctca ggacagcacc     120
   ctctgcctgt tcaccgactt tgactcccaa atcaatgtgc cgaaaaccat ggaatctgga     180
   acgttcatca ctgacaaaac tgtgctggac atgaaagcta tggattccaa gagcaatggg     240
   gccattgcct ggagcaacca gacaagcttc acctgccaag atatcttcaa agagaccaac     300
   gccacctacc ccagttcaga cgttccctgt gatgccacgt tgactgagaa aagctttgaa     360
   acagatatga acctaaactt tcaaaacctg tcagttatgg gactccgaat cctcctgctg     420
   aaagtagccg gatttaacct gctcatgacg ctgaggctgt ggtccagttg a              471
<210> 3
   <211> 435
   <212> PRT
   <213> artificial
<220>
   <223> mutated scTCR (Valpha-Linker-Vbeta-Cbeta) peptide
<400> 3
<210> 4
   <211> 156
   <212> PRT
   <213> artificial
<220>
   <223> mutated TCRalpha (Signal peptide-Calpha) peptide
<400> 4
   Met Glu Arg Leu Leu Cys Ser Leu Leu Gly Leu Leu Cys Thr Gln Val
   1               5                   10                  15
   Cys Trp Val Lys Gly Gln Asn Pro Glu Pro Ala Val Tyr Gln Leu Lys
               20                  25                  30
   Asp Pro Arg Ser Gln Asp Ser Thr Leu Cys Leu Phe Thr Asp Phe Asp
           35                  40                  45
   Ser Gln Ile Asn Val Pro Lys Thr Met Glu Ser Gly Thr Phe Ile Thr
       50                  55                  60
   Asp Lys Thr Val Leu Asp Met Lys Ala Met Asp Ser Lys Ser Asn Gly
   65                  70                  75                  80
   Ala Ile Ala Trp Ser Asn Gln Thr Ser Phe Thr Cys Gln Asp Ile Phe
                   85                  90                  95
   Lys Glu Thr Asn Ala Thr Tyr Pro Ser Ser Asp Val Pro Cys Asp Ala
               100                 105                 110
   Thr Leu Thr Glu Lys Ser Phe Glu Thr Asp Met Asn Leu Asn Phe Gln
           115                 120                 125
   Asn Leu Ser Val Met Gly Leu Arg Ile Leu Leu Leu Lys Val Ala Gly
       130                 135                 140
   Phe Asn Leu Leu Met Thr Leu Arg Leu Trp Ser Ser
   145                 150                 155